(19) Europäisches Patentamt
European Patent Office
Office européen des brevets

(11) Publication number: 0 172 031 B1

(12) **EUROPEAN PATENT SPECIFICATION**

(45) Date of publication of patent specification: **18.05.88**

(51) Int. Cl.[4]: **C 07 C 145/02,** C 07 D 333/22, C 07 D 307/52, C 07 D 213/53, A 01 N 47/04

(21) Application number: **85305805.5**

(22) Date of filing: **15.08.85**

(88) **A request pursuant to Rule 88 EPC for correction of the description and the claims was filed on 02.09.1985.**

# (54) Novel sulfenylated acylhydrazones, compositions containing them, methods of making them and their use.

(30) Priority: **17.08.84 US 641613**
**18.07.85 US 756160**

(43) Date of publication of application:
**19.02.86 Bulletin 86/08**

(45) Publication of the grant of the patent:
**18.05.88 Bulletin 88/20**

(84) Designated Contracting States:
**AT BE CH DE FR GB IT LI LU NL SE**

(56) References cited:
**CH-A-592 615**
**DE-A-2 838 750**

(73) Proprietor: **Rohm and Haas Company**
**Independence Mall West**
**Philadelphia, Pennsylvania 19105 (US)**

(72) Inventor: **Chi-Tung Hsu, Adam**
**1686 Heebner Way**
**Lansdale Pa. 19446 (US)**

(74) Representative: **Angell, David Whilton et al**
**ROHM AND HAAS (UK) LTD.**
**European Operations Patent Department**
**Lennig House 2 Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention is concerned with novel sulfenylated acylhydrazones, compositions containing them, methods of making them and their use in controlling living organisms.

The novel sulfenylated acylhydrazones of this invention are biocidally active compounds and, as such, are suitable for the control of living organisms and particularly microoganisms.

The sulfenylated acylhydrazones of this invention are represented by the formula

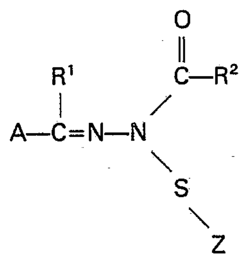

(I)

wherein

$R^1$ is hydrogen;
cyano;
straight or branched chain ($C_1$—$C_4$)-alkyl optionally substituted with imidazolyl, triazolyl or one or more of the same or different halo (chloro, fluoro, bromo or iodo); or
phenyl optionally substituted with one or more of the same of different halo (chloro, fluoro, bromo or iodo), nitro, cyano, ($C_1$—$C_4$)-alkyl, or ($C_1$—$C_4$)-alkoxy;

$R^2$ is hydrogen;
straight or branched chain ($C_1$—$C_{11}$)-alkyl optionally substituted with imidazolyl, triazolyl, or one or more of the same or different halo (chloro, fluoro, bromo or iodo);
($C_1$—$C_4$)-alkoxy;
($C_1$—$C_4$)-thioalkoxy;
($C_2$—$C_6$)-alkenyl optionally substituted with one or more of the same or different halo (chloro, fluoro, bromo or iodo);
heterocyclic optionally substituted with one or more of the same or different halo (chloro, fluoro, bromo or iodo), or nitro;
phenyl optionally substituted with one or more of the same or different halo (chloro, fluoro, bromo or iodo), ($C_1$—$C_4$)-alkyl, ($C_1$—$C_4$)-alkoxy or nitro; or
phenalkyl where the alkyl moiety contains one to four carbon atoms and the phenyl ring is optionally substituted with one or more of the same or different halo (chloro, fluoro, bromo or iodo), cyano, nitro, ($C_1$—$C_4$)-alkyl, ($C_1$—$C_4$)-alkoxy, or ($C_1$—$C_4$)-haloalkyl;

Z is ($C_1$—$C_4$)-haloalkyl; and

A is phenyl optionally substituted with one or more of the same or different halo (chloro, fluoro, bromo or iodo), nitro, cyano, hydroxy, ($C_1$—$C_6$)-alkyl, ($C_1$—$C_6$)-haloalkyl, ($C_1$—$C_6$)-thioalkyl, ($C_1$—$C_6$)-alkoxy, ($C_1$—$C_6$)-carbalkoxy, allyloxy, ($C_1$—$C_6$)-alkanoyloxy, ($C_1$—$C_6$)-alkylamino, ($C_1$—$C_6$)-dialkylamino or benzyloxy;
thienyl optionally substituted with one or more of the same or different nitro or halo (chloro, fluoro, bromo or iodo);
furyl optionally substituted with one or more of the same or different nitro or halo (chloro, fluoro, bromo or iodo);
pyridyl; or
naphthyl.

The term alkyl by itself or as a moiety of another substituent comprises a straight or branched chain alkyl group of the stated number of carbon atoms. The term "halo" includes chloro, fluoro, bromo and iodo. The term "haloalkyl" includes chloro, fluoro, bromo and iodo as mono, di, tri, tetra, penta or hexa substitutions (from one to six halo substituents) on an alkyl moiety. Examples of the alkyl portion include methyl, ethyl, propyl, isopropyl, butyl, isobutyl, sec-butyl, tert-butyl, and where indicated higher homologues such as pentyl, hexyl, heptyl, octyl and the like, together with their isomers. By analogy, "alkoxy" denotes methoxy, ethoxy, propoxy, isopropoxy, butoxy, sec-butoxy, isobutoxy, tert-butoxy and the like; "thioalkyl" denotes an —SR group where R is an alkyl moiety having the stated number of carbon atoms; "carbalkoxy" denotes a —$CO_2$R group where R is an alkyl moiety having the stated number of carbon atoms; "alkanoyloxy" denotes an —OCOR group where R is an alkyl moiety having the stated number of carbon atoms; "alkylamino" denotes an —NHR group where R is an alkyl moiety having the stated number of carbon atoms; and "dialkylamino" denotes a —NRR′ group where R and R′ are alkyl moieties, independently having the stated number of carbon atoms.

Heterocyclic comprises a ring containing three to five nuclear carbon atoms and one to three hetero atoms selected from nitrogen, oxygen and sulfur, such as furyl, thiophenyl, pyridyl, pyrrol, imidazolyl,

oxazolyl, thiazolyl, pyrazolyl, pyrimidyl and the like. By substituted phenyl is meant a phenyl ring having one or more hydrogen atoms on the ring replaced by another substituent.

Typical compounds within the scope of Formula I include the following:

4-Chlorobenzaldehyde methoxycarbonyl (trichloromethanesulfenyl) hydrazone,
2-Hydroxybenzaldehyde methoxycarbonyl (trichloromethanesulfenyl) hydrazone,
4-Hydroxybenzaldehyde methoxycarbonyl (trichloromethanesulfenyl) hydrazone,
4-Dimethylaminobenzaldehyde methoxycarbonyl (trichloromethanesulfenyl) hydrazone,
3,5-Dichlorobenzaldehyde methoxycarbonyl (trichloromethanesulfenyl) hydrazone,
4-Acetoxybenzaldehyde methoxycarbonyl (trichloromethanesulfenyl) hydrazone,
4-Methoxycarbonylbenzaldehyde methoxycarbonyl (trichloromethanesulfenyl) hydrazone.
2-Thiophenecarboxaldehyde methoxycarbonyl (trichloromethanesulfenyl) hydrazone,
5-Nitro-2-thiophenecarboxyaldehyde methoxycarbonyl (trichloromethanesulfenyl) hydrazone,
4-Methoxybenzaldehyde methoxycarbonyl (trichloromethanesulfenyl) hydrazone,
Benzaldehyde methoxycarbonyl (trichloromethanesulfenyl) hydrazone,
3-Methoxybenzaldehyde methoxycarbonyl (trichloromethanesulfenyl) hydrazone,
2-Methoxybenzaldehyde methoxycarbonyl (trichloromethanesulfenyl) hydrazone,
3-Bromobenzaldehyde methoxycarbonyl (trichloromethanesulfenyl) hydrazone,
2-Furaldehyde methoxycarbonyl (trichloromethanesulfenyl) hydrazone,
4-Bromobenzaldehyde methoxycarbonyl (trichloromethanesulfenyl) hydrazone,
4-Cyanobenzaldehyde methoxycarbonyl (trichloromethanesulfenyl) hydrazone,
4-Fluorobenzaldehyde methoxycarbonyl (trichloromethanesulfenyl) hydrazone,
2-Bromobenzaldehyde methoxycarbonyl (trichloromethanesulfenyl) hydrazone,
2-Fluorobenzaldehyde methoxycarbonyl (trichloromethanesulfenyl) hydrazone,
3-Nitrobenzaldehyde methoxycarbonyl (trichloromethanesulfenyl) hydrazone,
3-Fluorobenzaldehyde methoxycarbonyl (trichloromethanesulfenyl) hydrazone,
2,6-Dichlorobenzaldehyde methoxycarbonyl (trichloromethanesulfenyl) hydrazone,
2,4-Dichlorobenzaldehyde methoxycarbonyl (trichloromethanesulfenyl) hydrazone,
3,4-Dichlorobenzaldehyde methoxycarbonyl (trichloromethanesulfenyl) hydrazone,
4-Benzyloxybenzaldehyde methoxycarbonyl (trichloromethanesulfenyl) hydrazone,
2,4-Dimethoxybenzaldehyde methoxycarbonyl (trichloromethanesulfenyl) hydrazone,
3-Methylbenzaldehyde methoxycarbonyl (trichloromethanesulfenyl) hydrazone,
3,4,5-Trimethoxybenzaldehyde methoxycarbonyl (trichloromethanesulfenyl) hydrazone,
2-Chlorobenzaldehyde methoxycarbonyl (trichloromethanesulfenyl) hydrazone,
3-Chlorobenzaldehyde methoxycarbonyl (trichloromethanesulfenyl) hydrazone,
4-Chlorobenzaldehyde acetyl (trichloromethanesulfenyl) hydrazone,
4-Chlorobenzaldehyde benzoyl (trichloromethanesulfenyl) hydrazone,
Methyl-(5-nitro-2-thienyl) ketone methoxycarbonyl (trichloromethanesulfenyl) hydrazone,
4-Chlorobenzaldehyde chloroacetyl (trichloromethanesulfenyl) hydrazone,
Benzaldehyde chloroacetyl (trichloromethanesulfenyl) hydrazone,
2-Chlorobenzaldehyde chloroacetyl (trichloromethanesulfenyl) hydrazone,
alpha-chloroacetophenone methoxycarbonyl (trichloromethanesulfenyl) hydrazone,
4-Chlorobenzaldehyde formyl (trichloromethanesulfenyl) hydrazone,
2-Chlorobenzaldehyde formyl (trichloromethanesulfenyl) hydrazone,
4-Chlorobenzaldehyde ethoxycarbonyl (trichloromethanesulfenyl) hydrazone,
2-Chlorobenzaldehyde ethoxycarbonyl (trichloromethanesulfenyl) hydrazone,
Acetophenone methoxycarbonyl (trichloromethanesulfenyl) hydrazone,
4'-Chloroacetophenone methoxycarbonyl (trichloromethanesulfenyl) hydrazone,
4'-Chloropropiophenone methoxycarbonyl (trichloromethanesulfenyl) hydrazone,
4-Chlorobenzophenone methoxycarbonyl (trichloromethanesulfenyl) hydrazone, and
Benzophenone methoxycarbonyl (trichloromethanesulfenyl) hydrazone.

The sulfenylated acylhydrazones of this invention can be prepared by reacting a hydrazone of the following Formula

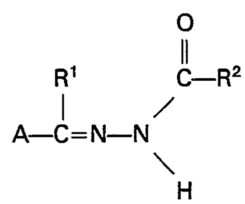

(II)

where A, $R^1$ and $R^2$ are as defined for Formula I in the presence of a base and a solvent or solvent mixture with a sulfenylating agent of the Formula

$$Hal—S—Z \quad (III)$$

where Hal is halogen, preferably chlorine, and Z is as defined for Formula I.

The above reaction is conducted using about an equimolar ratio of the reactants and base in the presence of an inert or substantially inert organic solvent or mixture thereof such as toluene, benzene, methylene chloride, chloroform, dioxane, tetrahydrofuran, dimethylformamide, dimethyl sulfoxide, and the like. Preferably, from about 1.0 to about 1.2 moles of the sulfenylating agents of Formula III and from about 1.0 to about 1.2 moles of base are used per mole of the hydrazones of Formula II. Suitable bases that may be used are inorganic and organic acid-binding compounds such as triethylamines, pyridine, sodium bicarbonate, potassium bicarbonate, and the like.

Generally these reactions can be carried out at temperatures in the range of from about 0°C to about 110°C. Preferably, the temperatures are in the range of from about 20°C to about 80°C.

The sulfenylated acylhydrazone product of Formula I is isolated by known methods.

Some typical examples of sulfenylating agents of Formula III which can be used in preparing the compounds of Formula I are: Trichloromethanesulfenyl-chloride, dichlorofluoromethanesulfenyl chloride, pentachloroethanesulfenyl chloride, and the like. These sulfenylating agents are generally commercially available or can be prepared by known methods

The hydrazone of formula II can be made by condensing a carbonyl compound of the formula

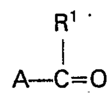

(IV)

where A and $R^1$ are as defined for Formula I with a monosubstituted carbonylhydrazide of the formula

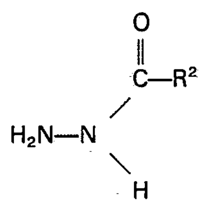

(V)

where $R^2$ is as defined for Formula I in the presence of an import solvent or solvent mixture at a temperature of from about 20°C to about 100°C. Typical inert solvents which may be used in this reaction include water, alcohols, ethyl acetate, dioxane, diethyl ether, dimethylformamide, dimethyl sulfoxide, and the like. Alcohols and aqueous alcohols are preferred solvents.

Examples of the carbonyl compounds of Formula IV that may be used in preparing the intermediate compounds of Formula II include: 4-chlorobenzaldehyde, 2-methoxybenzaldehyde, 2-furaldehyde, 5-nitro-2-thiophenecarbonaldehyde, methyl-(5-nitro-2-thienyl) ketone, O-chloroacetophenone, alpha-chloroacetophenone, alpha-imidazolylacetophenone, alpha-triazolylacetophenone, benzophenone, 1-naphthyl, and the like. These and other carbonyl compounds that may be used in the above reaction are generally commercially available or can be prepared by known procedures.

Examples of monosubstituted carbonyl hydrazides of Formula V that may be used in preparing the intermediate compounds of Formula II include: Methoxy-carbonylhydrazine, ethoxycarbonylhydrazine, chloroacetylhydrazine, benzoylhydrazine, 4-chlorobenzoylhydrazine, formylhydrazine, 2-furoic acid hydrazide, 2-thiophene carboxylic acid hydrazide, isonicotinic acid hydrazide and the like. These and other carbonyl hydrazides that may be used in the above reaction are generally commercially available or can be prepared by known procedures.

The following examples will further illustrate this invention but are not to be construed as limiting it in any way. In Table I, embodiments of compounds of Formula I prepared by the above-described process and constituting Examples 1 through 97 are set forth. Tables II lists the elemental analyses and melting points (or boiling ranges) for some examples previously described in Table I. All structures were confirmed by NMR analysis. Specific illustrative preparations of Examples 1, 2, 3, 5, 10, 11, 12, 18, 35, 47, 53, 60, 62, 77 and 94 are set forth after Table II.

TABLE I
Sulfenylated Acylhydrazone Examples

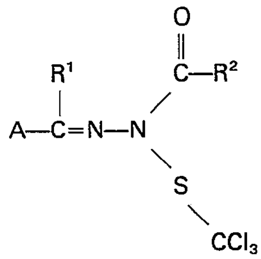

| Example No. | $R^1$ | $R^2$ | A |
|---|---|---|---|
| 1 | H | $OCH_3$ | $C_6H_4Cl$—4 |
| 2 | H | $C_6H_5$ | $C_6H_4Cl$—4 |
| 3 | H | $CH_2Cl$ | $C_6H_4Cl$—4 |
| 4 | H | $OCH_3$ | $C_6H_4OH$—2 |
| 5 | H | $OCH_3$ | $C_6H_4OH$—4 |
| 6 | H | $OCH_3$ | $C_6H_4N(CH_3)_2$—4 |
| 7 | H | $OCH_3$ | $C_6H_3Cl_2$—3,5 |
| 8 | H | $OCH_3$ | $C_6H_4OCOCH_3$—4 |
| 9 | H | $OCH_3$ | $C_6H_4CO_2CH_3$—4 |
| 10 | H | $CH_3$ | $C_6H_4Cl$—4 |
| 11 | H | $OCH_3$ | 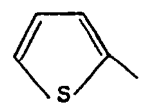 |
| 12 | H | $OCH_3$ | 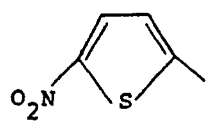 |
| 13 | H | $OCH_3$ | $C_6H_4OCH_3$—4 |
| 14 | H | $OCH_3$ | $C_6H_5$ |
| 15 | H | $OCH_3$ | $C_6H_4OCH_3$—3 |
| 16 | H | $OCH_3$ | $C_6H_4OCH_3$—2 |
| 17 | H | $OCH_3$ | $C_6H_4Br$—3 |
| 18 | H | $OCH_3$ | 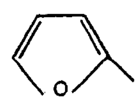 |
| 19 | H | $OCH_3$ | $C_6H_4Br$—4 |
| 20 | H | $OCH_3$ | $C_6H_4CN$—4 |
| 21 | H | $OCH_3$ | $C_6H_4F$—4 |
| 22 | H | $OCH_3$ | $C_6H_4Br$—2 |
| 23 | H | $OCH_3$ | $C_6H_4F$—2 |
| 24 | H | $OCH_3$ | $C_6H_4NO_2$—3 |
| 25 | H | $OCH_3$ | $C_6H_4F$—3 |
| 26 | H | $OCH_3$ | $C_6H_3Cl_2$—2,6 |
| 27 | H | $OCH_3$ | $C_6H_3Cl_2$—2,4 |
| 28 | H | $OCH_3$ | $C_6H_3Cl_2$—3,4 |
| 29 | H | $OCH_3$ | $C_6H_4(OCH_2C_6H_5)$—4 |
| 30 | H | $OCH_3$ | $C_6H_3(OCH_3)_2$—2,4 |
| 31 | H | $OCH_3$ | $C_6H_4CH_3$—3 |
| 32 | H | $OCH_3$ | $C_6H_2(OCH_3)_3$—3,4,5 |
| 33 | H | $OCH_3$ | $C_6H_4Cl$—2 |
| 34 | H | $OCH_3$ | $C_6H_4Cl$—3 |
| 35 | $CH_3$ | $OCH_3$ | 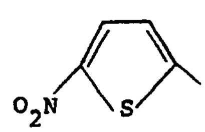 |
| 36 | $CH_2Cl$ | $OCH_3$ | $C_6H_5$ |

TABLE I (continued)

| Example No. | $R^1$ | $R^2$ | A |
|---|---|---|---|
| 37 | H | H | $C_6H_4Cl$—4 |
| 38 | H | H | $C_6H_4Cl$—2 |
| 39 | H | $OCH_2CH_3$ | $C_6H_4Cl$—4 |
| 40 | H | $OCH_2CH_3$ | $C_6H_4Cl$—2 |
| 41 | $CH_3$ | $OCH_3$ | $C_6H_5$ |
| 42 | $CH_3$ | $OCH_3$ | $C_6H_4Cl$—4 |
| 43 | $CH_2CH_3$ | $OCH_3$ | $C_6H_4Cl$—4 |
| 44 | $C_6H_5$ | $OCH_3$ | $C_6H_4Cl$—4 |
| 45 | $C_6H_5$ | $OCH_3$ | $C_6H_5$ |
| 46 | H | H | $C_6H_4CH_3$—2 |
| 47 | H | H | $C_6H_4CH_3$—4 |
| 48 | H | H | $C_6H_4CH_3$—3 |
| 49 | H | H | $C_6H_3Cl,F$—2,6 |
| 50 | H | H | $C_6H_3Cl_2$—2,6 |
| 51 | H | H | $C_6H_3Cl_2$—3,4 |
| 52 | H | H | $C_6H_3Cl_2$—3,5 |
| 53 | H | H | $C_6H_4CF_3$—4 |
| 54 | H | H | $C_6H_4Br$—4 |
| 55 | H | H | $C_6H_4Cl$—3 |
| 56 | H | H | $C_6H_4F$—2 |
| 57 | H | H | $C_6H_4F$—3 |
| 58 | H | H | $C_6H_4F$—4 |
| 59 | H | H | $C_6H_5$ |
| 60 | H | H | $C_6H_3Cl_2$—2,4 |
| 61 | H | H | $C_6H_4Br$—2 |
| 62 | H | H | $C_6H_4Br$—3 |
| 63 | H | H | $C_6H_3F_2$—2,6 |
| 64 | H | H | 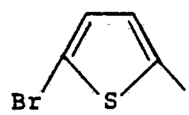 |
| 65 | H | H | $C_6H_4OCH_3$—2 |
| 66 | H | H | $C_6H_4OCH_3$—3 |
| 67 | H | H | $C_6H_4OCH_3$—4 |
| 68 | H | H | $C_6H_4NO_2$—3 |
| 69 | H | H | $C_6H_4SCH_3$—4 |
| 70 | H | H | $C_6H_3(CH_3)_2$—2,4 |
| 71 | H | H | $C_6H_3(CH_3)_2$—2,5 |
| 72 | H | H | $C_6H_4(O\overset{O}{C}—CH_3)$—4 |
| 73 | H | H | $C_6H_4OCH_2CH{=}CH_2$—4 |
| 74 | H | H | $C_6H_4CO_2CH_3$—4 |
| 75 | H | H | 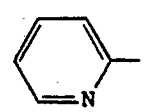 |
| 76 | H | H | 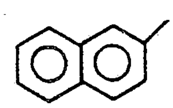 |
| 77 | H | $OCH_3$ | $C_6H_4CH_3$—4 |
| 78 | H | $OCH_3$ | $C_6H_4CF_3$—4 |
| 79 | H | $OCH_3$ | $C_6H_4CH_3$—2 |
| 80 | H | $OCH_3$ | $C_6H_4SCH_3$—4 |
| 81 | H | $OCH_3$ | $C_6H_3(CH_3)_2$—2,5 |
| 82 | H | $OCH_3$ | $C_6H_3F_2$—2,6 |
| 83 | H | $OCH_3$ | $C_6H_4OCH_2CH{=}CH_2$—4 |

TABLE I (continued)

| Example No. | $R^1$ | $R^2$ | A |
|---|---|---|---|
| 84 | H | $OCH_3$ | 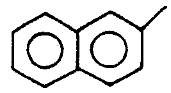 |
| 85 | H | $OCH_3$ | 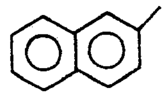 |
| 86 | H | $OCH_2CH_3$ | $C_6H_4CF_3$—4 |
| 87 | H | $OCH_2CH_3$ | $C_6H_3Cl,F$—2,6 |
| 88 | H | $OCH_2CH_3$ | $C_6H_4CH_3$—4 |
| 89 | H | $OCH_2CH_3$ | $C_6H_3Cl_2$—3,4 |
| 90 | H | $C_6H_4Cl$—4 | $C_6H_4Cl$—4 |
| 91 | H | $C_6H_4Cl$—2 | $C_6H_4Cl$—4 |
| 92 | H | $—CH_2C_6H_5$ | $C_6H_4CH_3$—2 |
| 93 | H | $C_6H_4Cl$—2 | $C_6H_4CH_3$—2 |
| 94 | H | $—O—C(CH_3)_3$ | $C_6H_4Cl$—4 |
| 95 | H | $—CH_2C_6H_5$ | $C_6H_4Cl$—4 |
| 96 | H | $—O—C(CH_3)_3$ | 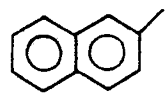 |
| 97 | H | 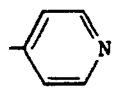 | $C_6H_4Cl$—4 |
| 98 | H | $CH_2Cl$ | $C_6H_4Cl$—2 |

TABLE II

| Example No. | Melting Point in °C | | Analysis C | H | N | S | Cl | Br | F |
|---|---|---|---|---|---|---|---|---|---|
| 1 | 110—112 | calc.* | 33.17 | 2.23 | 7.74 | 8.85 | 39.17 | | |
| | | found | 33.12 | 2.21 | 7.66 | 8.98 | 39.07 | | |
| 2 | 155—156 | calc. | 44.15 | 2.47 | 6.86 | 7.86 | 34.75 | | |
| | | found | 44.12 | 2.36 | 6.75 | 8.21 | 33.90 | | |
| 3 | 122—124 | calc. | 31.57 | 1.85 | 7.36 | 8.43 | 46.59 | | |
| | | found | 31.53 | 1.84 | 7.33 | 8.49 | 46.16 | | |
| 4 | 142—144 | calc. | 34.96 | 2.64 | 8.15 | 9.33 | 30.95 | | |
| | | found | 35.19 | 2.63 | 8.07 | 9.56 | 31.60 | | |
| 5 | 105—107 | | | | | | | | |
| 6 | 129—130 | calc. | 38.88 | 3.81 | 11.34 | 8.65 | 28.69 | | |
| | | found | 39.06 | 3.88 | 11.35 | 8.60 | 28.24 | | |
| 7 | 139—141 | calc. | 30.29 | 1.78 | 7.07 | 8.09 | 44.71 | | |
| | | found | 30.66 | 1.91 | 7.09 | 8.25 | 44.62 | | |
| 8 | 120—122 | calc. | 37.37 | 2.88 | 7.26 | 8.31 | 27.58 | | |
| | | found | 37.48 | 2.89 | 7.22 | 8.41 | 27.32 | | |
| 9 | 141—143 | calc. | 37.37 | 2.88 | 7.26 | 8.31 | 27.58 | | |
| | | douns | 37.48 | 2.89 | 7.26 | 8.51 | 27.54 | | |
| 10 | 114—118 | calc. | 34.71 | 2.33 | 8.10 | 9.26 | 40.98 | | |
| | | found | 34.97 | 2.35 | 8.14 | 8.91 | 40.74 | | |
| 11 | 80—82 | calc. | 28.80 | 2.11 | 8.40 | 19.22 | 31.88 | | |
| | | douns | 29.70 | 2.20 | 8.78 | 18.90 | 31.46 | | |
| 12 | 149—151 | calc. | 25.38 | 1.59 | 11.09 | 16.93 | 28.09 | | |
| | | found | 25.29 | 1.54 | 10.95 | 16.54 | 28.03 | | |
| 13 | 121—123 | calc. | 36.94 | 3.10 | 7.83 | 8.96 | 29.44 | | |
| | | found | 36.84 | 3.14 | 7.94 | 8.66 | 29.77 | | |
| 14 | 85—87 | | | | | | | | |
| 15 | 97—99 | calc. | 36.94 | 3.10 | 7.83 | 8.96 | 29.74 | | |
| | | found | 36.95 | 3.11 | 7.80 | 8.52 | 28.96 | | |
| 16 | 109—111 | calc. | 36.94 | 3.10 | 7.83 | 8.96 | 29.74 | | |
| | | found | 38.09 | 3.20 | 8.07 | 8.47 | 28.72 | | |
| 17 | 120—122 | calc. | 29.55 | 1.98 | 6.89 | 7.89 | 26.16 | 19.66 | |
| | | found | 29.64 | 1.97 | 6.81 | 7.80 | 25.38 | 18.13 | |
| 18 | 96—98 | | | | | | | | |
| 19 | 100—103 | calc. | 29.55 | 1.98 | 6.89 | 7.89 | 26.16 | 17.66 | |
| | | found | 29.62 | 1.97 | 6.80 | 7.75 | 25.25 | 17.97 | |
| 20 | 150—153 | calc. | 37.47 | 2.29 | 11.92 | 9.09 | 30.16 | | |
| | | found | 37.72 | 2.33 | 11.89 | 8.78 | 24.55 | | |
| 21 | 138—140 | calc. | 34.75 | 2.33 | 8.11 | 9.28 | 30.78 | | |
| | | found | 34.91 | 2.29 | 8.09 | 9.14 | 30.98 | | |

TABLE II (continued)

| Example No. | Melting Point in °C | | Analysis C | H | N | S | Cl | Br | F |
|---|---|---|---|---|---|---|---|---|---|
| 22 | 121—123 | calc. | 29.55 | 1.98 | 6.89 | 7.89 | 26.16 | 19.66 | |
| | | found | 29.71 | 1.96 | 6.85 | 7.85 | 25.00 | 18.19 | |
| 23 | 108—110 | calc. | 34.75 | 2.33 | 8.11 | 9.28 | 30.78 | | 5.49 |
| | | found | 34.92 | 2.28 | 8.05 | 9.53 | 29.53 | | 5.43 |
| 24 | 153—155 | calc. | 32.23 | 2.16 | 11.28 | 8.60 | 28.54 | | |
| | | found | 32.51 | 2.15 | 11.27 | 8.53 | 28.48 | | |
| 25 | 103—105 | calc. | 34.75 | 2.33 | 8.11 | 9.28 | 30.78 | | 5.49 |
| | | found | 34.76 | 2.32 | 8.08 | 9.07 | 30.98 | | 5.82 |
| 26 | 84—86 | calc. | 30.29 | 1.78 | 7.06 | 8.09 | 44.71 | | |
| | | found | 30.68 | 1.78 | 7.13 | 8.13 | 44.81 | | |
| 27 | 132—134 | calc. | 30.29 | 1.78 | 7.06 | 8.09 | 44.71 | | |
| | | found | 30.68 | 1.74 | 7.00 | 8.03 | 44.62 | | |
| 28 | 123—125 | calc. | 30.29 | 1.78 | 7.06 | 8.09 | 44.71 | | |
| | | found | 30.28 | 1.72 | 7.12 | 8.20 | 44.63 | | |
| 29 | 123—124 | calc. | 47.08 | 3.49 | 6.46 | 7.39 | 24.52 | | |
| | | found | 47.21 | 3.47 | 6.42 | 7.46 | 24.60 | | |
| 30 | 84—88 | calc. | 37.18 | 3.38 | 7.23 | 8.27 | 27.44 | | |
| | | found | 37.50 | 3.47 | 7.26 | 7.57 | 26.01 | | |
| 31 | 139—141 | calc. | 38.67 | 3.25 | 8.20 | 9.38 | 31.13 | | |
| | | found | 38.84 | 3.23 | 8.25 | 9.29 | 31.18 | | |
| 32 | 96—98 | calc. | 37.38 | 3.62 | 6.71 | 7.68 | 25.46 | | |
| | | found | 37.86 | 3.71 | 6.59 | 7.64 | 25.37 | | |
| 33 | 123—125 | calc. | 33.17 | 2.23 | 7.74 | 8.85 | 39.17 | | |
| | | found | 33.58 | 2.21 | 7.81 | 8.98 | 39.40 | | |
| 34 | 117—119 | calc. | 33.17 | 2.23 | 7.74 | 8.85 | 39.17 | | |
| | | found | 33.42 | 2.23 | 7.64 | 8.78 | 39.18 | | |
| 35 | 117—120 | calc. | 27.53 | 2.05 | 10.70 | 16.33 | 27.09 | | |
| | | found | 27.81 | 2.11 | 10.14 | 21.08 | 26.75 | | |
| 36 | Oil | | | | | | | | |
| 37 | 105—109 | calc. | 32.56 | 1.82 | 8.44 | 9.66 | 42.71 | | |
| | | found | 32.68 | 2.02 | 8.50 | 9.91 | 43.48 | | |
| 38 | 81—84 | calc. | 32.56 | 1.82 | 8.44 | 9.66 | 42.71 | | |
| | | found | 32.69 | 2.00 | 8.40 | 9.91 | 43.40 | | |
| 39 | 87—89 | | | | | | | | |
| 40 | 96—99 | calc. | 35.13 | 2.68 | 7.45 | 8.52 | 37.00 | | |
| | | found | 35.65 | 2.76 | 7.53 | 8.74 | 37.81 | | |
| 41 | Oil | | | | | | | | |
| 42 | Oil | | | | | | | | |

TABLE II (continued)

| Example No. | Melting Point in °C | | Analysis C | H | N | S | Cl | Br | F |
|---|---|---|---|---|---|---|---|---|---|
| 43 | Oil | | | | | | | | |
| 44 | | | | | | | | | |
| 45 | | | | | | | | | |
| 46 | 63—65 | calc. | 38.54 | 2.92 | 8.99 | 10.29 | 34.13 | | |
| | | found | 39.11 | 2.91 | 9.07 | 10.37 | 33.71 | | |
| 47 | 102—104 | calc. | 38.54 | 2.92 | 8.99 | 10.29 | 34.13 | | |
| | | found | 38.77 | 2.77 | 9.03 | 10.60 | 33.75 | | |
| 48 | 83—85 | calc. | 38.54 | 2.92 | 8.99 | 10.29 | 34.13 | | |
| | | found | 38.77 | 2.88 | 9.10 | 10.49 | 33.92 | | |
| 49 | 101—104 | calc. | 30.89 | 1.44 | 8.01 | 9.16 | 40.51 | | |
| | | found | 30.84 | 1.32 | 8.00 | 9.18 | 40.71 | | |
| 50 | 111—115 | calc. | 29.49 | 1.38 | 7.65 | 8.75 | 48.37 | | |
| | | found | 29.40 | 1.31 | 7.55 | 8.93 | 48.04 | | |
| 51 | 110—113 | calc. | 29.49 | 1.38 | 7.65 | 8.75 | 48.37 | | |
| | | found | 29.67 | 1.39 | 7.55 | 8.96 | 48.45 | | |
| 52 | 149—152 | calc. | 29.49 | 1.38 | 7.65 | 8.75 | 48.37 | | |
| | | found | 29.80 | 1.34 | 7.62 | 8.70 | 47.32 | | |
| 53 | 86—93 | calc. | 32.85 | 1.66 | 7.66 | 8.77 | 29.09 | | |
| | | found | 32.83 | 1.77 | 7.63 | 8.73 | 29.17 | | |
| 54 | 119—123 | calc. | | | | | | | |
| | | found | | | | | | | |
| 55 | 76—80 | calc. | 32.55 | 1.83 | 8.44 | 9.66 | 42.71 | | |
| | | found | 32.56 | 1.78 | 8.49 | 9.56 | 42.67 | | |
| 56 | 82—87 | calc. | 34.25 | 1.92 | 8.88 | 10.20 | 33.70 | | |
| | | found | 34.44 | 1.89 | 8.96 | 10.20 | 33.85 | | |
| 57 | 64—68 | calc. | 34.25 | 1.92 | 8.88 | 10.20 | 33.70 | | |
| | | found | 34.48 | 1.87 | 9.06 | 9.98 | 33.83 | | |
| 58 | 86—89 | calc. | 34.25 | 1.92 | 8.88 | 10.20 | 33.70 | | |
| | | found | 33.72 | 1.79 | 8.97 | 9.88 | 33.72 | | |
| 59 | 74—77 | calc. | 36.32 | 2.38 | 9.42 | 10.80 | 35.74 | | |
| | | found | 36.71 | 2.42 | 9.90 | 10.27 | 34.63 | | |
| 60 | 102—105 | calc. | 29.49 | 1.38 | 7.65 | 8.75 | 48.37 | | |
| | | found | 30.99 | 1.73 | 8.26 | 7.17 | 43.04 | | |
| 61 | 80—82 | calc. | | | | | | | |
| | | found | | | | | | | |
| 62 | 78—82 | calc. | | | | | | | |
| | | found | | | | | | | |

TABLE II (continued)

| Example No. | Melting Point in °C | | Analysis C | H | N | S | Cl | Br | F |
|---|---|---|---|---|---|---|---|---|---|
| 63 | 95—100 | calc. | 32.40 | 1.51 | 8.40 | 9.61 | 31.88 | | |
| | | found | 32.49 | 1.56 | 8.41 | 9.44 | 31.92 | | |
| 64 | 75—78 | calc. | | | | | | | |
| | | found | | | | | | | |
| 65 | 69—73 | calc. | 36.66 | 2.77 | 8.55 | 9.79 | 32.46 | | |
| | | found | 36.87 | 2.86 | 8.57 | 9.88 | 32.75 | | |
| 66 | 93—96 | calc. | 36.66 | 2.77 | 8.55 | 9.79 | 32.46 | | |
| | | found | 36.82 | 2.84 | 8.70 | 9.80 | 32.17 | | |
| 67 | 86—89 | calc. | 36.66 | 2.77 | 8.55 | 9.79 | 32.46 | | |
| | | found | 37.58 | 2.95 | 8.61 | 9.82 | 31.68 | | |
| 68 | 98—104 | calc. | 31.55 | 1.77 | 12.27 | 9.36 | 31.04 | | |
| | | found | 31.78 | 1.82 | 12.37 | 9.33 | 30.88 | | |
| 69 | 103—106 | calc. | | | | | | | |
| | | found | | | | | | | |
| 70 | 84—86 | calc. | 40.57 | 3.41 | 8.60 | 9.84 | 32.66 | | |
| | | found | 40.71 | 2.79 | 8.62 | 10.07 | 32.91 | | |
| 71 | 90—94 | calc. | 40.57 | 3.41 | 8.60 | 9.84 | 32.66 | | |
| | | found | 40.93 | 2.98 | 8.76 | 10.15 | 31.04 | | |
| 72 | 106—108 | calc. | 37.15 | 2.56 | 7.88 | 9.01 | 29.90 | | |
| | | found | 37.41 | 2.60 | 7.87 | 9.10 | 29.82 | | |
| 73 | | | | | | | | | |
| 74 | 106—109 | calc. | 37.15 | 2.56 | 7.88 | 9.01 | 29.90 | | |
| | | found | 37.36 | 2.54 | 7.90 | 9.06 | 29.85 | | |
| 75 | 104—109 | calc. | 32.18 | 2.03 | 14.08 | 10.74 | 35.62 | | |
| | | found | 32.16 | 2.03 | 14.11 | 11.00 | 35.95 | | |
| 76 | 132—136 | calc. | 44.91 | 2.61 | 8.06 | 9.22 | 30.59 | | |
| | | found | 45.30 | 2.54 | 7.98 | 9.39 | 30.12 | | |
| 77 | 118—119 | calc. | 38.67 | 3.25 | 8.20 | 9.38 | 31.13 | | |
| | | found | 38.76 | 3.29 | 8.10 | 9.59 | 31.07 | | |
| 78 | 101—103 | calc. | 33.40 | 2.04 | 7.08 | 8.10 | 26.88 | | |
| | | found | 33.55 | 2.20 | 7.29 | 8.20 | 26.38 | | |
| 79 | 109—110 | calc. | 40.52 | 3.69 | 7.88 | 9.01 | 29.90 | | |
| | | found | 38.38 | 3.26 | 8.36 | 9.61 | 31.05 | | |
| 80 | 107—109 | calc. | 35.35 | 2.97 | 7.50 | 17.16 | 28.46 | | |
| | | found | 34.98 | 2.98 | 7.41 | 17.29 | 27.91 | | |
| 81 | 131—132 | calc. | 40.52 | 3.69 | 7.88 | 9.01 | 29.90 | | |
| | | found | 39.99 | 3.68 | 7.89 | 9.16 | 29.99 | | |
| 82 | 89—92 | calc. | 33.03 | 1.94 | 7.71 | 8.82 | 29.25 | | |
| | | found | 33.08 | 2.12 | 7.95 | 9.17 | 29.39 | | |

TABLE II (continued)

| Example No. | Melting Point in °C | | Analysis C | H | N | S | Cl | Br | F |
|---|---|---|---|---|---|---|---|---|---|
| 83 | 82—83 | calc. | 42.46 | 3.57 | 7.62 | 8.72 | 28.92 | | |
| | | found | 40.41 | 3.45 | 8.07 | 8.70 | 28.17 | | |
| 84 | | | | | | | | | |
| 85 | | | | | | | | | |
| 86 | 49—51 | calc. | 35.18 | 2.47 | 6.84 | 7.83 | 25.96 | | |
| | | found | 35.29 | 2.54 | 7.16 | 8.17 | 25.45 | | |
| 87 | 79—81 | calc. | 33.52 | 2.31 | 7.11 | 8.14 | 35.98 | | |
| | | found | 33.47 | 2.41 | 7.11 | 8.37 | 36.25 | | |
| 88 | 106—108 | calc. | | | | | | | |
| | | found | | | | | | | |
| 89 | 82—89 | calc. | 32.18 | 2.21 | 6.83 | 7.81 | 43.18 | | |
| | | found | 32.32 | 2.22 | 6.92 | 8.19 | 42.94 | | |
| 90 | 148—167 | calc. | | | | | | | |
| | | found | | | | | | | |
| 91 | 101—105 | calc. | 40.71 | 2.05 | 6.33 | 7.24 | 40.05 | | |
| | | found | 40.98 | 2.10 | 6.29 | 7.53 | 40.20 | | |
| 92 | 98—102 | calc. | 50.82 | 3.77 | 6.97 | 7.98 | 26.47 | | |
| | | found | 50.72 | 3.78 | 6.96 | 8.50 | 27.07 | | |
| 93 | Oil | | | | | | | | |
| 94 | 87—89 | calc. | 38.63 | 3.50 | 6.93 | 7.93 | 35.09 | | |
| | | found | 37.95 | 3.44 | 8.10 | 8.74 | 35.91 | | |
| 95 | 95—97 | calc. | 45.52 | 2.87 | 6.64 | 7.59 | 33.59 | | |
| | | found | 45.81 | 3.11 | 7.48 | 7.67 | 33.48 | | |
| 96 | 109—118 | calc. | 48.64 | 4.09 | 6.68 | 7.64 | 25.34 | | |
| | | found | 48.38 | 3.91 | 6.91 | 8.37 | 25.43 | | |
| 97 | | | | | | | | | |

*calculated

Example 1

Preparation of 4-chlorobenzaldehyde methoxycarbonyl (trichloromethane sulfenyl) hydrazone

A 125 ml, 3-necked, round-bottomed flask was equipped with a paddle stirrer. The flask was charged with 4-chlorobenzaldehyde-(N'-methoxycarbonyl)-hydrazone (5.0 g, 23.53 mmole) and toluene (60 ml, without further drying). Triethylamine (3.47 ml, 25 mmole, distilled from sodium hydroxide NaOH) or just dried over NaOH, was added in one portion. To the above suspension was added dropwise the trichloromethanesulfenyl chloride ($ClSCCl_3$) (46.65 g, 25 mmole) from a syringe at room temperature with vigorous stirring. The reaction was slightly exothermic. The reaction mixture was stirrer at room temperature for 1 to 2 hours. The reaction mixture was poured into a mixture of ethyl acetate (60 ml) and water (60 ml). The organic layer was subsequently washed with 5% $NaHCO_3$ solution (50 ml), $H_2O$ (2 × 50 ml) and saturated NaCl solution (50 ml). After drying over $MgSO_4$ and filtering, the filtrate was concentrated on a rotary evaporator and further dried under vacuum to give the product (7.24 g, yield 86%).

Example 2

Preparation of 4-Chlorobenzaldehyde benzoyl (trichloromethanesulfenyl) hydrazone

Following the procedures of Example 1, 4-chlorobenzaldehyde-(N'-benzoyl)-hydrazone (2.6 g, 10 mmole) in toluene (20 ml) was treated with $Et_3N$ (1.21 g, 12 mmole) and $ClSCCl_3$ (2.1 g, 12 mmole) to obtain 3.3 g (80% yield) of product as fine needles.

Example 3

Preparation of 4-Chlorobenzaldehyde chloroacetyl (trichloromethanesulfenyl) hydrazone

To a suspension of 4-chlorobenzaldehyde-N'-chloroacetylhydrazone (2.8 g, 12.12 mmole) in dry toluene (25 ml) and dry DMF (10 ml) at room temperature under $N_2$ was slowly added pyridine (1 ml, 13 mmole, distilled from NaOH) and subsequently $ClSCCl_3$ (1.4 ml, 13 mmole).

After addition, the reaction was heated at 40°C to 50°C for 60 minutes. The cooled reaction mixture was diluted with EtOAc (50 ml) and washed with $H_2O$ (2 × 50 ml) and brine (50 ml). The organic layer was dried over $Na_2SO_4$. After filtering off the drying agent, the solvents were removed under reduced pressure to give a brown residue. The pure product was obtained by column chromatography on silica gel by eluting with $CHCl_3$, 3.11 g (67%).

Example 5

Preparation of 4-Hydroxybenzaldehyde methoxycarbonyl (trichloromethanesulfenyl) hydrazone

A suspension of 4-hydroxybenzaldehyde-(N'-methoxycarbonyl)-hydrazone (3 g, 15.5 mmole) of toluene (70 ml, distilled from $CaH_2$) at room temperature under $N_2$ with magnetic stirring was treated with 2 equivalents of $Et_3N$ (triethylamine) (3.125 g, 31 mmole) and then 2 equivalents of trichloromethanesulfenyl chloride (5.8 g, 31 mmole) was added dropwise over 5 minutes.

The reaction mixture was heated at 60—70°C for 100 minutes, then cooled to room temperature and quenched with 50% saturated NaCl solution (100 ml). The aqueous phase was extracted with EtOAc (2 × 100 ml). The combined organic layer was washed with $H_2O$ (2 × 100 ml) and brine. The solution was dried over $MgSO_4$ and evaporated on a rotary evaporator to give a residue which was triturated with EtOAc-hexane (1:9).

The solid was filtered and the filtrate was evaporated to dryness. The solid (4.0 g) obtained from filtrate was crystallized from EtOAc-hexane (1:4) to give the desired product (1.06 g, yield 20%).

Example 10

Preparation of 4-Chlorobenzaldehyde acetyl (trichloromethanesulfenyl) hydrazone

Following the procedures described for Example 1, 4-chlorobenzaldehyde-N'-acetyl-hydrazone (3g, 15.3 mmole) in toluene (60 ml) was treated with $Et_3N$ (1.56 g, 16 mmole) and $ClSCCl_3$ (2.9 g, 16 mmole) to obtain 5.2 g (98% yield) of product.

Example 11

Preparation of 2-Thiophenecarboxyaldehyde methoxycarbonyl (trichloromethanesulfenyl) hydrazone

Following the procedures of Example 1, 2-thiophenecarboxaldehyde-[N'-methoxycarbonyl)-hydrazone (5 g, 27.2 mmole) was treated with $Et_3N$ (2.83 g, 28 mmole) and subsequently $ClSCCl_3$ (5.21 g, 28 mmole) to obtain 8.46 g (95% yield) of crystalline product.

Example 12

Preparation of 5-Nitro-2-thiophenecarboxaldehyde methoxycarbonyl (trichloromethanesulfenyl) hydrazone

To a suspension of 5-nitro-2-thiophenecarboxaldehyde(N'-methoxycarbonyl) hydrazone (2 g, 8.73 mmole) in toluene (30 ml) and DMF (2 ml, dried over NaOH) at room temperature under $N_2$ with stirring was added $Et_3N$ (0.91 g, 9 mmole) followed by slow addition of $ClSCCl_3$ (1.674 g, 9 mmole). After addition, the reaction mixture was stirred at room temperature for 2 hours and heated at 60°C for 1 hour. The cooled reaction mixture was diluted with EtOAc (100 ml) and washed subsequently with $H_2O$ (50 ml), 5% $NaHCO_3$ (50 ml), $H_2O$ (50 ml), and saturated NaCl. The organic layer was dried over $MgSO_4$ and evaporated to dryness. The residue was triturated with $CHCl_3$ and filtered to remove the unreacted starting material. The filtrate was concentrated and crystallized from EtOAc-hexane (1:1) to give 1.8 g (60% yield) of product as yellow needles.

Example 18

Preparation of 2-Furaldehyde methoxycarbonyl (trichloromethanesulfenyl) hydrazone

Following the procedures described for Example 1, 4 g (23.8 mmole) of 2-Furaldehyde hydrazone was treated with $Et_3N$ (2.26 g, 25 mmole) and $ClSCCl_3$ (4.65 g, 25 mmole) to obtain 7.18 g (yield 95%) of crude product.

Example 35

Preparation of Methyl-(5-nitro-2-thienyl)ketone methoxycarbonyl(trichloromethanesulfenyl) hydrazone

Following the procedures of Example 1, methyl-(5-nitro-2-thienyl) ketone methoxycarbonylhydrazone

(1.5 g, 6.17 mmole) was treated with $Et_3N$ (1 ml, 7 mmole) and $ClSCCl_3$ (0.71 g, 6.5 mmole) to obtain a crude residue. The pure product (0.8 g, yield 33%) was obtained by silica gel column chromatography by elution with $CHCl_3$.

Example 47

Preparation of 4-Methylbenzaldehyde formyl (trichloromethanesulfenyl) hydrazone

To a mixture of 4-methylbenzaldehyde formic acid hydrazone (5.0 g, 30.86 mmole) and trichloromethanesulfenyl chloride (6.31 g, 33.95 mmole) in dry toluene (50 ml) at room temperature under nitrogen was added slowly the triethylamine (3.43 g, 33.95 mmole) with a mechanical stirrer. The reaction was slightly exothermic. The reaction mixture was stirred at room temperature for an additional one hour. The reaction mixture was then diluted with 100 ml of ethyl acetate, washed with water and saturated NaCl solution, and dried over magnesium sulfate. After filtering off the drying agent the filtrate was concentrated on a rotary evaporator to give a light yellow residue. A pure product (5.98 g, yield 62.2%) was obtained by crystallization from hexane.

Example 53

Preparation of 4-Trifluoromethylbenzaldehyde formyl (trichloromethanesulfenyl) hydrazone

Following the procedures described in Example 47, 4-trifluoromethylbenzaldehyde formic acid hydrazone (5.0 g, 23.1 mmole) and trichloromethanesulfenyl chloride (4.74 g, 25.46 mmole) in dry toluene (50 ml) was treated with triethylamine (2.57 g, 25.46 mmole) to obtain 5.1 g (60.3% yield) of pure product.

Example 60

Preparation of 2,4-Dichlorobenzaldehyde formyl (trichloromethanesulfenyl) hydrazone

Following the procedures described in Example 47, 2,4-dichlorobenzaldehyde hydrazone (5.0 g, 23.04 mmole) in toluene (50 ml) was treated with trichloromethanesulfenylchloride (5.14 g, 27.65 mmole) and pyridine (2.18 g, 27.65 mmole) to obtain 3.9 g (46.2% yield) of product.

Example 62

Preparation of 3-Bromobenzaldehyde formyl (trichloromethanesulfenyl) hydrazone

Following the procedures described in Example 47, 4-bromobenzaldehyde formic acid hydrazone (5.0 g, 22.03 mmole) in toluene (50 ml) was treated with trichloromethanesulfenyl chloride (4.92 g, 26.43 mmole) and pyridine (2.09 g, 26.43 mmole) to obtain 5.0 g (60.3% yield) of nearly pure product.

Example 77

Preparation of 4-Methylbenzaldehyde methoxycarbonyl (trichloromethanesulfenyl) hydrazone

Following the procedures described in Example 1, 4-methylbenzaldehyde methoxycarbonyl hydrazone (3.0 g, 15.63 mmole) in dry toluene (50 ml) was treated with triethylamine (1.89 g, 18.75 mmole) and trichloromethane sulfenyl chloride (3.49 g, 18.75 mmole) to obtain 3.0 g (56.2% yield) of pure product.

Example 94

Preparation of 4-Chlorobenzaldehyde 5-butoxycarbonyl (trichloromethanesulfenyl) hydrazone

To a mixture of 4-chlorobenzaldehyde t-butoxycarbonylhydrazone (5.0 g, 19.65 mmole) and triethylamine (2.38 g, 23.58 mmole) in dry toluene (50 ml) at room temperature under nitrogen was added dropwise the trichloromethanesulfenyl chloride (4.39 g, 23.58 mmole) with stirring. After addition the reaction mixture was stirred for an additional 1.5 hours at room temperature. The reaction mixture was then diluted with ethyl acetate, washed with water and saturated NaCl solution, and dried over magnesium sulfate. After filtering off the drying agent, the filtrate was concentrated on a rotary evaporator to give a residue. The pure product (3.0 g, 31.8%) was obtained by crystallization from hexane.

The novel sulfenylated acylhydrazone compounds of this invention exhibit biocidal activity and as such are suitable for use in the control of living organisms and particularly microorganisms.

Primarily because of their activity, sulfenylated acylhydrazones preferred as biocides are those compounds of Formula I where:

$R^1$ is hydrogen, methyl or chloromethyl;

$R^2$ is hydrogen, methoxy or ethoxy;

A is phenyl substituted with one, two or three of the same or different chloro, fluoro, bromo, nitro, trifluoromethyl, methyl, methoxy or carbmethoxy or bromothienyl; and

Z is trichloromethyl.

Antibacterial and fungicidal activity were evaluated by the Serial Dilution Test (Broth Titer Test) wherein a series of broths containing varying dilutions of a test compound are halved starting with 500 ppm. Following dilution, the broth tubes containing 225 microliters are inoculated with 25 microliters of inoculum containing approximately 1 × $10^6$ colony forming units of test organism per milliliter. These tubes are then incubated and observed for growth. The values obtained, which are shown in Table III, rounded off to the nearest whole number, represent the minimum concentration in parts per million at which the compound under evaluation renders complete control of this organism. The following fungi and bacteria were employed in this test:

| Fungi | Bacteria |
|---|---|
| *Aspergillis niger* (ANIG) | *Escherichia coli* (ECOL) |
| *Aureobasidium pullulans* (APUL) | *Pseudomonas aeruginosa* (PSAE) |
| *Candida albicans* (CALB) | *Pseudomonas fluorescens* (PSFL) |
| | *Staphylococcus aureus* (SAUR) |

TABLE III

Microbiocidal Activity[1]

| Ex. No. | Fungistatic Evaluation | | | Bacteriostatic Evaluation | | | |
|---|---|---|---|---|---|---|---|
| | ANIG | APUL | CALB | ECOL | PSAE | PSFL | SAUR |
| 1 | 500 | 63 | 63 | >500 | >500 | >500 | 500 |
| 2 | >500 | —[2] | — | — | — | >500 | — |
| 3 | >500 | — | — | — | — | >500 | — |
| 4 | 250 | — | — | — | — | >500 | — |
| 5 | 500 | — | — | — | — | >500 | — |
| 6 | >500 | — | — | — | — | >500 | — |
| 7 | >500 | — | — | — | — | >500 | — |
| 8 | 31—125 | 63 | 63 | — | — | >500 | >500 |
| 9 | 16—500 | 16—31 | 8—16 | >500 | >500 | >500 | >500 |
| 10 | 250—500 | — | — | — | — | >500 | — |
| 11 | 500 | — | — | — | — | >500 | — |
| 12 | >500 | — | — | — | — | >500 | — |
| 13 | >500 | — | — | — | — | >500 | — |
| 14 | 500 | — | — | — | — | >500 | — |
| 15 | 125—250 | 31—63 | 125 | >500 | >500 | >500 | 250—500 |
| 16 | >500 | — | — | — | — | 500 | — |
| 17 | >500 | — | — | — | — | 500 | — |
| 18 | >500 | — | — | — | — | 500 | — |
| 19 | 63—125 | 125 | 63—125 | >500 | >500 | >500 | >500 |
| 20 | 250—500 | — | — | — | — | >500 | — |
| 21 | >500 | — | — | — | — | >500 | — |
| 22 | 250—500 | — | — | — | — | >500 | — |
| 23 | 31—125 | 63—125 | 63 | >500 | >500 | >500 | 250—500 |
| 24 | 250—500 | 31 | 8—16 | >500 | >500 | >500 | >500 |
| 25 | 250—500 | 63—125 | 63—125 | >500 | >500 | >500 | 250 |
| 26 | 31—63 | 31 | 31 | >500 | >500 | >500 | >500 |
| 27 | >500 | — | — | — | — | >500 | — |
| 28 | 500 | — | — | — | — | >500 | — |
| 29 | >500 | — | — | — | — | >500 | — |
| 30 | 125—500 | 250 | >500 | >500 | >500 | >500 | >500 |
| 31 | 500 | — | — | — | — | >500 | — |
| 32 | >500 | — | — | — | — | >500 | — |
| 33 | 16—33 | 16—31 | 16 | >500 | >500 | >500 | >500 |
| 34 | 125—500 | 63 | 63 | >500 | >500 | >500 | >500 |
| 35 | 125—250 | 31 | >500 | 250 | >500 | >500 | 125 |
| 36 | 31 | — | — | — | — | >500 | — |
| 37 | 8 | 8 | >500 | >500 | >500 | >500 | 125 |
| 38 | 8 | 8 | 125 | >400 | >500 | >500 | 125 |
| 39 | 63 | — | — | — | — | >500 | — |
| 40 | 500 | — | — | — | — | >500 | — |
| 41 | 250—500 | — | — | — | — | >500 | — |
| 42 | 250—500 | — | — | — | — | >500 | — |
| 43 | ≥500 | — | — | — | — | >500 | — |
| 44 | 125 | — | — | — | — | >500 | — |
| 45 | 250 | — | — | — | — | >500 | — |
| 46 | 125 | — | — | — | — | >500 | — |
| 47 | 16 | 8 | 63 | >500 | >500 | 250 | >500 |
| 48 | >500 | — | — | — | — | >500 | — |
| 49 | 8 | 8 | <4 | >500 | >500 | >500 | >500 |
| 50 | 31 | 8 | <4 | >500 | >500 | >500 | >500 |
| 51 | 125 | — | — | — | — | >500 | — |
| 52 | 250 | — | — | — | — | >500 | — |

TABLE III (continued)

Microbiocidal Activity[1]

| Ex. No. | Fungistatic Evaluation | | | Bacteriostatic Evaluation | | | |
|---|---|---|---|---|---|---|---|
| | ANIG | APUL | CALB | ECOL | PSAE | PSFL | SAUR |
| 53 | 16 | 8 | 8 | >500 | >500 | >500 | >500 |
| 54 | 63 | 16 | 31 | >500 | >500 | >500 | 125 |
| 55 | 16 | 8 | 8 | >500 | >500 | >500 | >500 |
| 56 | 31 | 31 | 31 | >500 | >500 | >500 | 250 |
| 57 | 125 | — | — | — | — | >500 | — |
| 58 | 250 | — | — | — | — | >500 | — |
| 59 | 125 | — | — | — | — | 250 | — |
| 60 | 16 | 8 | 8 | >500 | >500 | >500 | 63 |
| 61 | 125 | — | — | — | — | >500 | — |
| 62 | 16 | 16 | 16 | >500 | >500 | >500 | 63 |
| 63 | 31 | 8 | 31 | >500 | >500 | >500 | >500 |
| 64 | 16 | 16 | 8 | >500 | >500 | >500 | 250 |
| 65 | 31 | 31 | 31 | >500 | >500 | >500 | 63 |
| 66 | 16 | 63 | 63 | >500 | >500 | >500 | 125 |
| 67 | 31 | 31 | 31 | >500 | >500 | >500 | 125 |
| 68 | 125 | — | — | — | — | >500 | — |
| 69 | 125 | — | — | — | — | >500 | — |
| 70 | 16 | 8 | 31 | >500 | >500 | >500 | 31 |
| 71 | 63 | 8 | 16 | >500 | >500 | >500 | >500 |
| 72 | 250 | — | — | — | — | >500 | — |
| 73 | 125 | — | — | — | — | >500 | — |
| 74 | >500 | — | — | — | — | >500 | — |
| 75 | 250 | — | — | — | — | >500 | — |
| 76 | 125 | 8 | 31 | >500 | >500 | >500 | >500 |
| 77 | >500 | — | — | — | — | >500 | — |
| 78 | 250 | — | — | — | — | >500 | — |
| 79 | 500 | — | — | — | — | >500 | — |
| 80 | 500 | — | — | — | — | >500 | — |
| 81 | >500 | — | — | — | — | >500 | — |
| 82 | 500 | — | — | — | — | >500 | — |
| 83 | 500 | — | — | — | — | >500 | — |
| 84 | >500 | — | — | — | — | >500 | — |
| 85 | >500 | — | — | — | — | >500 | — |
| 86 | >500 | — | — | — | — | >500 | — |
| 87 | 125 | — | — | — | — | >500 | — |
| 88 | >500 | — | — | — | — | >500 | — |
| 89 | 16 | 63 | 63 | >500 | >500 | >500 | >500 |
| 90 | >500 | — | — | — | — | >500 | — |
| 91 | >500 | — | — | — | — | >500 | — |
| 92 | 250 | — | — | — | — | >500 | — |
| 93 | >500 | — | — | — | — | >500 | — |
| 94 | >500 | — | — | — | — | >500 | — |
| 95 | >500 | — | — | — | — | >500 | — |
| 96 | >500 | — | — | — | — | >500 | — |
| 97 | >500 | — | — | — | — | >500 | — |
| 98 | 63—250 | 250 | 16 | >500 | >500 | >500 | 250 |

[1]Reported as minimum inhibitory concentration in parts per million.
[2]No data reported.

The sulfenylated acylhydrazones of this invention also exhibit activity against assorted phytopathogenic fungi.

Primarily because of their activity, sulfenylated acylhydrazones preferred as fungicides are those compounds of Formula I where

$R^1$ is hydrogen;

$R^2$ is hydrogen, methoxy or chlorophenyl;

A is phenyl substituted with one, two or three of the same or different chloro, fluoro, bromo, cyano, nitro, carbmethoxy or acetoxy; and

Z is trichloromethyl.

In evaluating the compounds of this invention, potted test plants in proper condition of growth for susceptibility to the fungal disease are sprayed to run-off with suspensions of the compounds to be evaluated in a dosage series on a moving belt at a carrier volume of about 1403.1 liters/hectare (150 gallons/acre). The sprayed plants are allowed to dry. The proper plants are then inoculated with the fungal spores which are allowed to incubate until the disease has developed. Plants are visually inspected and the percent disease control (as compared to inoculated control) estimated.

The following specific test methods were employed in evaluating the fungicidal activity of the compounds of this invention.

Example A

Wheat Stem Rust (Puccinia graminis) F. sp. tritici race 15B—2

*Method 1*:

Seven-day-old wheat plants (var. "Wanser") are trimmed to approximately 6.35 cm (2.5 inches), 24 hours prior to chemical application to provide a uniform plant height and to facilitate uniform inoculation. Wheat stem rust is cultured on wheat seedlings (var. "Wanser") for a period of 14 days under existing greenhouse conditions. A spore suspension of wheat stem rust is prepared by harvesting infected leaves and shaking the leaves vigorously with water plus a surfactant. The spore suspension is filtered through cheesecloth to remove debris and adjusted to three to five spores per large square on a hemacytometer. Wheat plants are inoculated by applying the stem rust spore suspension, until run-off, with a DeVilbiss atomizer at 34.48 kPa (5 psi) air pressure. After inoculation, the plants are placed into a humid environment at approximately 20°C (68°F). A timer is used to permit 12 hours of continuous darkness followed by a minimum of 3 to 4 hours of light with an intensity of 5380 lx (500 foot candles). The temperature in the chamber should not exceed 29.4°C (85°F). At the end of the light period, the plants are placed into a greenhouse environment. The plants are permitted to grow under greenhouse conditions for a period of 2 weeks prior to making treatment comparisons. Wheat stem rust is characterized by brick red spores in irregularly shaped sori in the leaves and stems of the wheat seedings.

*Method 2*:

Substantially the same procedures as described above for Method 1 are followed except wheat plants and seedlings (var. "Tyler") are used and the wheat plants are inoculated with a spore suspension prepared by harvesting the spores from infected plants with a vacuum pump or rust collector and adding to a spray oil at a concentration of four mg of spores to one ml of oil. The inoculum is then dispensed into gelatin capsules and applied with a vacuum pump. Plants are allowed to dry for about twenty minutes and then placed in a humid environment.

Example B

Wheat Powdery Mildew (Erysiphe graminis f. sp. tritici)

"Pennoll" wheat seedlings (7—14 days old) are trimmed 24 hours prior to chemical application to provide a uniform plant height and to facilitate uniform inoculation.

Wheat powdery mildew is cultured on wheat seedlings in a controlled temperature room at 18.3—23.9°C (65—75°C).

Mildew spores are shaken from culture plants onto seedlings. Inoculated seedlings are kept in the controlled temperature room and sub-irrigated. Disease control is rated 7—10 days after inoculation.

Example C

Wheat Leaf Rust (Puccinia recondita) F. sp. tritici

*Method 1*:

Wheat leaf rust is cultured on wheat seedlings (var. "Pennoll") for a period of 10 days under existing greenhouse conditions. Seven-day-old wheat plants (var. "Pennoll") treated with the test compound are inoculated by applying a leaf rust spore suspension, until run-off, with a hand held plant mister. After inoculation, the plants are placed into existing greenhouse conditions (humid environment with the temperature in the chamber not exceeding 29.4°C (85°F)). The plants are permitted to grow for a period of 8—10 days prior to making treatment comparisons. Wheat leaf rust is characterized in orange spores in regularly shaped sori on the leaves of the wheat seedlings.

*Method 2*:

Substantially, the same procedures described for Example A, Method 2 are followed except after inoculation, the plants are handled as described above for wheat leaf rust, Method 1.

Example D

Cucumber Downy Mildew (Pseudoperonospora cubensis)

Cucumber (var. "Marketer") seedlings are grown for three weeks at 18.3—23.9°C (65—75°F) in moderate light before use.

*Pseudoperonospora cubensis* is cultured on cucumber seedlings for 7 days at 18.3—23.9°C (65—75°F) in moderate light (alternating light and dark periods). Spores are harvested by adding deionized water and shaking leaves in a quart jar. The spore suspension is filtered through cheesecloth to remove plant debris and adjusted to a concentration of about 20,000 spores per ml using a hemacytometer.

The cucumber plants are inoculated by spraying the under side of the leaves with a DeVilbiss atomizer until small droplets are observed on the leaves. The inoculated leaves are incubated in a mist chamber for 24 hours at 21.1°C (70°F) and then subsequently incubated for 6—7 days in a controlled temperature room under mist at 18.3—23.9°C (65—75°C).

Treatment comparisons are made 7 days after inoculation by estimating percent infected leaf surface. Symptoms appear as yellowing of the upper leaf surface and grey sporulating areas on the lower leaf surface.

Example E

Tomato Late Blight (Phytophthora infestans)

Tomato (var. "Rutgers") seedlings, 7.62 to 10.16 cm (3 to 4 inches) tall, are fertilized with a water soluble fertilizer 4 to 5 days prior to chemical application to promote rapid succulent growth and better sympton expression. A spore suspension is prepared by collecting leaves from tomato plants (var. "Pixie") that are sporulating; placed them in a quart jar; adding about 100 ml of water; recapping the jar and shaking it vigorously; filtering the liquid through one layer of cheesecloth; and adjusting the suspension to between 10,000 and 20,000 spores per ml. The spore suspension is applied with a DeVilbiss atomizer at 55.16 to 68.95 kPa (8 to 10 psi) air pressure onto the leaf undersurface until fine droplets are formed. Inoculated seedlings are placed in a humid environment at 15.6°—16.7°C (60°—62°F). for 40 to 45 hours, prior to being placed in the greenhouse at 21.1°—23.9°C (70°—75°F). Treatment comparisons are made 4 to 6 days after inoculation. Tomato late blight symptoms appear as irregular, water-soaked patches which enlarge and become brown. Severely infected plants will have no healthy leaf tissue and white sporangia covering most or all of the plants.

Example F

Rice Sheat Blight (Pellicularia filamentosa f. sp. sasakii)

*Method 1*:

Rice seedlings 10—14 days old (var. "Lebonnet") are trimmed to a uniform height of 10.16—12.7 cm(4—5 inches) 24 hours prior to chemical application.

*Pellicularia filamentosa* f. sp. *sasakii* is cultured on an autoclaved mixture of crushed rice seeds/potato dextrose broth (100 g/30 ml) in a 500 ml wide-mouth Erlenmeyer flask. Ten-day-old cultures are run through a blender to produce uniform inoculum. Approximately one teaspoon of inoculum is spread among the rice seedlings on the soil surface of each pot (7.62 cm (3") diameter). Inoculated seedlings are incubated for two to five days in a humidity cabinet (29.4—32.2°C/85—90°F). Treatment comparisons are made immediately after removing the seedlings from the cabinet. The disease appears as a white furry or web-like mycelial growth extending from the soil surface up the stems of the seedlings.

*Method 2*:

Seedlings of rice cultivar "M—201" are grown in the greenhouse at 20—30°C in 5.08 cm (2-inch) pots in unsterilized soil for 14 days. Prior to the application of chemicals, the plants are trimmed with scissors to a height of 10.16—12.7 cm (4—5 inches).

Inoculum is produced in shake culture using the following procedure: autoclaved 500 ml wide-mouth flasks containing 150 ml potato dextrose broth are inoculated with a small piece of mycelium or a single sclerotium of Rhizoctonia solani Kuhn. The flasks are placed on an electric shaker (1500 rpm) at 22°C and a photo period of 14—16 hours for 6 days. A slurry containing 100 ml deionized water, 20 g rice flour (no additives) and 23 g mycelium (wet weight) is prepared in a blender in the appropriate quantity to inoculate 5.08 cm (2 inch) pots with 4 ml/pot. Blend the mixture for about 1 min.

The slurry is dispensed into 5.08 cm (2 inch) pots using pipettes with an oversized opening at 4 ml/pot (10 ml/7.62 cm (3 inch) pot). While dispensing the inoculum, the pot should be tilted to insure uniform distribution of the slurry over the entire soil surface. During the inoculation the slurry is kept in suspension using a stirring plate at medium speed.

Plants are put in a humidity cabinet at 28°C for about 43 hours and then kept in a humidity cabinet at 25°C for 53 hours (photo period for both cases 16 hours). The height of mycelial growth is observed as compared to the inoculated control plants.

Example G

Cucumber Anthracnose (Colletotrichum lagenarium

Cucumber (var. "Marketer") seedlings are 14 days old when treated.

*Collectrichum lagenarium* is cultured on green bean agar (GBA) in petri plates for 7 days under dark conditions. Petri plates of GBA are inoculated by spreading inoculum over medium with an inoculating needle. Spores are harvested from plates by adding deionized water to the GBA plates. The agar surface is scraped with a rubber policeman or similar blunt object. The spore suspension is filtered through

cheesecloth to remove mycelial and agar fragments and then adjusted to a concentration of 1—2 × $10^5$ spores per ml.

Treated cucumber plants are inoculated by spraying the leaves (especially the underside) until a uniform film of inoculum is observed on the plant. Inoculated plants are incubated in a humid environment at 21.1°—23.9°C (70°—75°F). for 72 hours. They are removed from the humid environment, allowed to dry, and placed under existing greenhouse conditions.

Treatment comparisons are made 10—14 days after inoculation. Typical anthracnose symptoms are small spots on the foliage that begin as small yellowish or water-soaked areas that enlarge rapidly and become necrotic.

Example H

Barley Net Blotch (Helminthosporium teres)

Barley plants (var. "Pennrad") are trimmed to a height of approximately 7.62 cm (3 inches) 24 hours prior to chemical application. This procedure provides plants of a uniform height and permits rapid inoculation of treated plants. The barley plants are inoculated by spraying the foliage of the plants with a hand sprayer until small droplets of the inoculum are observed on the leaves. Inoculated plants are incubated in a humid environment at 23.9°—29.4°C (75°—85°F) for 24 hours prior to being placed in the greenhouse at 21.1°—23.9°C (70°—75°F). Treatment comparisons are made 6 to 7 days after inoculation. Typical barley net blotch symptoms initially appear as irregular sunken water-soaked areas which become necrotic as the lesions enlarge.

Example I

Rice Blast (Piricularia oryzae)

*Method 1*:

Rice plants (var. "Calrose") are trimmed to a height of approximately 12.7 cm (5 inches) 24 hours prior to chemical application. This procedure provides plants of uniform height and permits rapid inoculation of treated plants. Rice plants are inoculated by spraying the leaves and stems with a hand sprayer until a uniform film of inoculum is observed on the leaves. The inoculated plants are incubated in a humid environment (23.9°—29.4°C/75°—85°F.) for 48 hours prior to being placed in a greenhouse environment. Treatment comparisons are made 7 to 8 days after inoculation. Initial rice blast lesions appear as small brown necrotic spots on the foliage. The typical lesions is eliptical, 1 to 2 cm. long with a large necrotic gray center and brown margins.

*Method 2*:

Seedlings of the rice cultivar "M—201" are grown in the greenhouse at 20—30°C in 5.08 cm (2-inch) pots containing unsterilized soil for 14 days. Rice plants are not trimmed before use.

Inoculum is produced in vitro on oatmeal agar (50 g Gerber baby oatmeal, 20 g bacto agar, 10 g bacto dextrose, 100 ml deionized water). The plates are inoculated with a mycelial plug (7—14 days old) of *Piricularia oryzae*. The outer edge of the dark region is used in the transfer. Inoculated plates are maintained at room temperature under constant fluorescent light.

*P. oryzae* plates 10—14 days old are flooded with a solution containing: 0.25 g sodium oleate, 2 g gelatin, 1000 ml dionized water. The plates are scraped with a rubber policeman to release conidia, filter through a double layer of cheesecloth spore suspension adjusted to 25,000—30,000 spores/ml using a hemacytometer.

The spore suspension is sprayed on opposite sides of a double row of rice plants using a hand sprayer. Sufficient inoculum should be applied to achieve uniform distribution from soil to tip of rice leaves on opposite sides of each pot (approx. 50 ml/50 pots). Shake the son-of-a-gun after each pass to keep solution in suspension.

Inoculated plants are immediately placed in a humidity cabinet at 25°C for about 66 hours prior to moving them to the greenhouse. Plants are subirrigated but not allowed to stand in water more than 2 hours.

After about 76 hours under greenhouse conditions the plants are observed and the treatment comparisons are made.

Example J

Chocolate Spot of English Broad Bean (Botrytis fabae)

*Botrytis fabae* is cultured on potato dextrose agar in petri plates for 14 days at room temperature in the dark. The petri plates are flooded with a mix of water and apple juice (2:1 by volume) and the conidia are scraped off the culture surface into the liquid. A test compound dissolved in 2:1:1 water:acetone:methanol is applied to run-off by a mechanical overhead sprayer onto three week old *Vicia faba* (English broad bean) plants and the plants allowed to dry. The conidial suspension is misted onto the plants. Treated and inoculated plants are kept in a 21.1°C (70°F) mist chamber under low light for 3 days. The disease is rated by counting the number of lesions per plant.

Where more than one test method is stated, the second method (Method 2) was used for the evaluations set forth in Table IV at 100 ppm.

TABLE IV

Phytopathogenic Fungi Activity

| Ex. No. | Rate[1] (ppm) | WSR[2] | WPM | CDM | TLB | RSB | CA | BH | RB | BOT | WLR |
|---|---|---|---|---|---|---|---|---|---|---|---|
| 1 | 300 | 98 | 96 | 99 | 85 | 0 | 0 | –[3] | 65 | – | – |
| 2 | 300 | 85 | 75 | 70 | 0 | 0 | 0 | – | 10 | – | – |
| 3 | 300 | 96 | 93 | 100 | 90 | 0 | 50 | – | 0 | – | – |
| 4 | 300 | 25 | 30 | 60 | 30 | 0 | 0 | – | 100 | – | – |
| 5 | 300 | 75 | 0 | 30 | 0 | 0 | 0 | – | 99 | – | – |
| 6 | 300 | 35 | 0 | 100 | 0 | 0 | 0 | – | 90 | – | – |
| 7 | 300 | 100 | 0 | 0 | 0 | 0 | 0 | – | 100 | – | – |
| 8 | 300 | 95 | 0 | 0 | 0 | 0 | 75 | – | 98 | – | – |
| 9 | 300 | 98 | 0 | 50 | 0 | 0 | 0 | – | 90 | – | – |
| 10 | 150 | – | 40 | 0 | 0 | 0 | 40 | 0 | 0 | 0 | 0 |
| 11 | 150 | – | 0 | 0 | 0 | 0 | 87 | 0 | 100 | 34 | 75 |
| 12 | 150 | – | 40 | 0 | 0 | 0 | 60 | 70 | 86 | 64 | 80 |
| 13 | 150 | – | 0 | 30 | 30 | 0 | 90 | 0 | 84 | 0 | 90 |
| 14 | 150 | – | 40 | 30 | 0 | 0 | 95 | 0 | 100 | 0 | 99 |
| 15 | 150 | – | 60 | 86 | 40 | 0 | 90 | 50 | 86 | 76 | 99 |
| 16 | 150 | – | 0 | 0 | 0 | 0 | 0 | 0 | 64 | 0 | 80 |
| 17 | 150 | – | 0 | 76 | 0 | 0 | 100 | 70 | 64 | 0 | 90 |
| 18 | 150 | – | 0 | 0 | 0 | 0 | 0 | 0 | 44 | 46 | 0 |
| 19 | 150 | – | 98 | 25 | 40 | 0 | 100 | 42 | 98 | 98 | 98 |
| 20 | 150 | – | 0 | 30 | 20 | 0 | 100 | 56 | 70 | 96 | 92 |
| 21 | 150 | – | 50 | 78 | 10 | 0 | 98 | 72 | 0 | 98 | 99 |
| 22 | 150 | – | 0 | 75 | 30 | 0 | 96 | 0 | 75 | 90 | 99 |
| 23 | 150 | – | 40 | 75 | 50 | 0 | 100 | 40 | 0 | 70 | 100 |
| 24 | 150 | – | 0 | 0 | 50 | 0 | 84 | 24 | 0 | 70 | 85 |
| 25 | 150 | – | 60 | 35 | 100 | 0 | 66 | 70 | 40 | 80 | 97 |
| 26 | 150 | – | 45 | 97 | 80 | 0 | 98 | 54 | 80 | 85 | 91 |
| 27 | 150 | – | 60 | 10 | 0 | 0 | 88 | 74 | 45 | 70 | 93 |
| 28 | 150 | – | 0 | 0 | 10 | 0 | 98 | 80 | 95 | 80 | 97 |
| 29 | 150 | – | 40 | 0 | 0 | 0 | 80 | 32 | 0 | 70 | 45 |
| 30 | 150 | – | 0 | 0 | 0 | 0 | 80 | 0 | 0 | 0 | 72 |
| 31 | 150 | – | 50 | 20 | 0 | 0 | 100 | 82 | 0 | 83 | 97 |
| 32 | 150 | – | 50 | 0 | 0 | 0 | 62 | 10 | 0 | 0 | 93 |
| 33 | 150 | – | 70 | 60 | 0 | 0 | 70 | 88 | 90 | 60 | 96 |
| 34 | 150 | – | 0 | 99 | 30 | 0 | 98 | 100 | 0 | 80 | 98 |
| 35 | 150 | – | 60 | 10 | 0 | 0 | 94 | 70 | 0 | 40 | 90 |
| 36 | 300 | 90 | 0 | 0 | 0 | 0[4] | – | – | 0 | – | 50[4] |
| 37 | 300 | 100 | 30 | 0 | 80 | 0 | – | – | 50 | – | – |
| 38 | 300 | 100 | 60 | 95 | 0 | – | – | – | 50 | – | – |
| 39 | 300 | 100 | 50 | 95 | 100 | – | – | – | 80 | – | – |
| 40 | 300 | 0 | 50 | 100 | 95 | – | – | – | 50 | – | – |
| 41 | 100 | – | 0 | 0 | 0 | 0 | – | – | 0 | – | 50 |
| 42 | 100 | – | 50 | 0 | 0 | 0 | – | – | 50 | – | 50 |
| 43 | – | – | – | – | – | – | – | – | – | – | – |
| 44 | 100 | – | 0 | 0 | 0 | 0 | – | – | 0 | – | 99 |
| 45 | 100 | – | 0 | 0 | 50 | 0 | – | – | 0 | – | 100 |
| 46 | 100 | – | 0 | 0 | 0 | 0 | – | – | 0 | – | 0 |
| 47 | 100 | – | 50 | 0 | 0 | 0 | – | – | 0 | – | 50 |
| 48 | 100 | – | 50 | 0 | 0 | 0 | – | – | 0 | – | 0 |
| 49 | 100 | – | 59 | 50 | 0 | 0 | – | – | 0 | – | 0 |
| 50 | 100 | – | 0 | 50 | 0 | 0 | – | – | 0 | – | 75 |
| 51 | 100 | – | 75 | 70 | 0 | 0 | – | – | 0 | – | 75 |
| 52 | 100 | – | 75 | 50 | 0 | 0 | – | – | 0 | – | 50 |
| 53 | 100 | – | 50 | 50 | 0 | 0 | – | – | 0 | – | 0 |
| 54 | 100 | – | 50 | 50 | 0 | 0 | – | – | 0 | – | 0 |
| 55 | 100 | – | 0 | 0 | 0 | 0 | – | – | 0 | – | 50 |
| 56 | 100 | – | 0 | 50 | 0 | 0 | – | – | 0 | – | 50 |
| 57 | 100 | – | 0 | 0 | 0 | 0 | – | – | 0 | – | 50 |
| 58 | 100 | – | 0 | 0 | 0 | 0 | – | – | 0 | – | 0 |

TABLE IV (continued)

Phytopathogenic Fungi Activity

| Ex. No. | Rate[1] (ppm) | WSR[2] | WPM | CDM | TLB | RSB | CA | BH | RB | BOT | WLR |
|---|---|---|---|---|---|---|---|---|---|---|---|
| 59 | 100 | – | 0 | 0 | 0 | 0 | – | – | 0 | – | 0 |
| 60 | 100 | – | 0 | 0 | 0 | 0 | – | – | 0 | – | 0 |
| 61 | 100 | – | 0 | 0 | 0 | 0 | – | – | 0 | – | 50 |
| 62 | 100 | – | 0 | 0 | 0 | 0 | – | – | 0 | – | 0 |
| 63 | 100 | – | 0 | 0 | 0 | 0 | – | – | 0 | – | 0 |
| 64 | 100 | – | 0 | 0 | 0 | 0 | – | – | 0 | – | 50 |
| 65 | 100 | – | – | – | – | – | – | – | – | – | – |
| 66 | 100 | – | – | – | – | – | – | – | – | – | – |
| 67 | 100 | – | – | – | – | – | – | – | – | – | – |
| 68 | 100 | – | – | – | – | – | – | – | – | – | – |
| 69 | 100 | – | – | – | – | – | – | – | – | – | – |
| 70 | 100 | – | – | – | – | – | – | – | – | – | – |
| 71 | 100 | – | – | – | – | – | – | – | – | – | – |
| 72 | 100 | – | – | – | – | – | – | – | – | – | – |
| 73 | 100 | – | – | – | – | – | – | – | – | – | – |
| 74 | 100 | – | – | – | – | – | – | – | – | – | – |
| 75 | 100 | – | – | – | – | – | – | – | – | – | – |
| 76 | 100 | – | – | – | – | – | – | – | – | – | – |
| 77 | 100 | – | 75 | 99 | 80 | 0 | – | – | 95 | – | 95 |
| 78 | 100 | – | 50 | 85 | 0 | 0 | – | – | 0 | – | 50 |
| 79 | 100 | – | 0 | 100 | 0 | 0 | – | – | 80 | – | 75 |
| 80 | 100 | – | 0 | 50 | 0 | 0 | – | – | 80 | – | 50 |
| 81 | 100 | – | 0 | 70 | 0 | 0 | – | – | 0 | – | 50 |
| 82 | 100 | – | 0 | 85 | 0 | 0 | – | – | 80 | – | 75 |
| 83 | 100 | – | 50 | 0 | 0 | 0 | – | – | 50 | – | 75 |
| 84 | 100 | – | 80 | 0 | 0 | 0 | – | – | 0 | – | 0 |
| 85 | 100 | – | 0 | 0 | 0 | 50 | – | – | 0 | – | 50 |
| 86 | 100 | – | 0 | 70 | 0 | 0 | – | – | 0 | – | 50 |
| 87 | 100 | – | 75 | 100 | 50 | 0 | – | – | 70 | – | 0 |
| 88 | 100 | – | – | – | – | – | – | – | – | – | – |
| 89 | 100 | – | – | – | – | – | – | – | – | – | – |
| 90 | 100 | – | – | – | – | – | – | – | – | – | – |
| 91 | 100 | – | – | – | – | – | – | – | – | – | – |
| 92 | 100 | – | – | – | – | – | – | – | – | – | – |
| 93 | 100 | – | – | – | – | – | – | – | – | – | – |
| 94 | 100 | – | 75 | 99 | 0 | 0 | – | – | 50 | – | 75 |
| 95 | 100 | – | 75 | 0 | 0 | 0 | – | – | 0 | – | 0 |
| 96 | 100 | – | – | – | – | – | – | – | – | – | – |
| 97 | 100 | – | 0 | 0 | 0 | 50 | – | – | 0 | – | 75 |

[1]Concentration in parts per million (ppm) at which compound tested.
[2]WSR = Wheat Stem Rust
WPM = Wheat Powdery Mildew
CDM = Cucumber Downy Mildew
TLB = Tomato Late Blight
RSB = Rice Sheath Blight
CA = Cucumber Anthracnose
BH = Barley Net Blotch
RB = Rice Blast
BOT = Chocolate Spot of English Broad Bean
WLR = Wheat Leaf Rust
[3]No Data Reported
[4]Evaluated at 100 ppm

Generally, control of a microorganism is achieved in accordance with the invention by contacting the organism with a sulfenylated acylhydrazone in an amount which is effective to control said organism. Any of the techniques known in the art can be employed to disseminate the compounds of this invention in a manner so as to achieve the desired contact with the organism to be controlled. Spraying and fumigating are typical of such techniques.

The compounds of this invention can be readily utilized as bactericides and fungicides or combinations thereof in any locus, such as, for example, paper pulp processes, cooling towers, aqueous polymer dispersions, water-based paints, seed treatment applications and the like. In addition, these compounds and compositions containing them can function as, for example, fabric or leather preservatives, wood preservatives, cosmetic preservatives, soap additives, sanitizing agents, such as in laundry soaps and detergents, and preservatives for metal working compounds, such as emulsifiable cutting oils, preservatives for fuels, fiber spin finish biocides and the like.

In general, a locus subject to contamination by microorganisms can be protected in accordance with this invention by incorporating into the locus a sulfenylated acylhydrazone in an amount which is effective to control said microorganisms. The term "contamination" is meant to include any attack by microorganisms which leads to a chemical or physical breakdown or disintegration of the locus as well as the proliferation of the microorganisms within the locus without an accompanying deleterious effect. The exact amount of sulfenylated acylhydrazone required will, of course, vary with the medium being controlled, the particular sulfenylated acylhydrazone or compositions containing the sulfenylated acylhydrazone being employed and other factors. Typically, excellent control is obtained when the sulfenylated acylhydrazones are incorporated in the range of 0.1 to 10,000 parts per million (ppm) or up to about 95% based on the weight of the composition. A range of 0.5 to 2,500 ppm is preferred.

The term "control" as employed in the specification and claims of this application is to be construed as the effect of any means which adversely affects the existence or growth of any living organism or microorganism. This effect may comprise a complete killing action, eradication, arresting in growth, inhibition, reduction in number or any combination thereof.

The sulfenylated acylhydrazones of this invention are especially useful as agricultural fungicides. As such, they are particularly valuable when formulated in a fungicidal composition. Such compositions normally comprise an agronomically acceptable carrier and a sulfenylated acylhydrazone or mixture of sulfenylated acylhydrazones as the active agent. Where necessary or desirable, surfactants or other additives may be incorporated to give uniformly formulated mixtures. By "agronomically acceptable carrier" is meant any substance which be utilized to dissolve, dispense or diffuse the chemical incorporated therein without impairing the effectiveness of the toxic agent and which does no permanent damage to such environment as soil, equipment and agronomic crops.

For use as agricultural fungicides, the compounds of this invention are usually taken up in an agronomically acceptable carrier or formulated so as to render them suitable for subsequent dissemination. For example the sulfenylated acylhydrazone can be formulated as wettable powders, emulsion concentrates, dusts, granular formulations, aerosols or flowable emulsifiable concentrates. In such formulations, the sulfenylated acylhydrazones are extended with a liquid or solid carrier and, when desired, suitable surfactants are likewise incorporated.

Certain compounds of this invention can be dissolved in a water-miscible liquid, such as dimethylformamide, dimethylsulfoxide, acetone, aromatic hydrocarbons and the like. Such solutions are easily extended with water.

The sulfenylated acylhydrazones can be taken up on or mixed with a finely particled solid carrier as, for example, clays, inorganic silicates, carbonates, and silicas. Organic carriers can also be employed. Dust concentrates are commonly made wherein sulfenylated acylhydrazones are present in the range of from about 20% to about 80% by weight. For ultimate applications, these concentrates are normally extended with an additional solid to give an active ingredient content of from about 1% to about 20%.

Wettable powder formulations are made by incorporating the compounds of this invention in an inert, finely divided solid carrier along with a surfactant which may be one or more emulsifying, wetting, dispersing or spreading agents or blend of these. The sulfenylated acylhydrazones are usually present in the range of from about 10% to about 80% by weight and the surfactants from about 0.5% to about 10% by weight. Commonly used emulsifying and wetting agents include polyoxyethylated derivatives of alkylphenols, fatty alcohols, fatty acids, and alkylamines, alkylarene sulfonates and dialkyl sulfosuccinates. Spreading agents include such materials as glycerol mannitan laurate and a condensate of polyglycerol and oleic acid modified with phthalic anhydride. Dispersing agents include such materials as the sodium salt of the copolymer of maleic anhydride and an olefin such as diisobutylene, sodium lignin sulfonate and sodium formaldehydenaphthalene sulfonates.

One convenient method for preparing a solid formulation is to impregnate the sulfenylated acylhydrazone toxicant onto the solid carrier by means of a volatile solvent, such as acetone. In this manner adjuvants, such as activators, adhesives, plant nutrients, synergists and various surfactants, can also be incorporated.

Emulsifiable concentrate formulations can be prepared by dissolving the sulfenylated acylhydrazones of this invention in an agronomically acceptable organic solvent and adding a solvent-soluble emulsifying agent. Suitable solvents are usually water-immiscible and may be found in the hydrocarbon, chlorinated

hydrocarbon, ketone, ester, alcohol and amide classes of organic solvents. Mixtures of solvents are commonly employed. The surfactants useful as emulsifying agents may constitute from about 0.5% to about 10% by weight of the emulsifiable concentrate and may be anionic, cationic or non-ionic in character. Anionic surfactants include alcohol sulfates or sulfonates, alkylarene sulfonates and sulfosuccinates. Cationic surfactants include fatty acid alkylamine salts and fatty acid alkyl quaternaries. Nonionic emulsifying agents include alkylene oxide adducts of alkylphenols, fatty alcohols, mercaptans and fatty acids. The concentration of the active ingredients may vary from about 10% to about 80% by weight, preferably in the range of about 25% to about 50%.

For use as phytopathogenic agents, these compounds should be applied in an effective amount sufficient to exert the desired biocidal activity by techniques well-known in the art. Usually, this will involve the application of the sulfenylated acylhydrazones to the locus to be protected in an effective amount when incorporated in an agronomically acceptable carrier. However, in certain situations, it may be desirable and advantageous to apply the compounds directly onto the locus to be protected without the benefit of any substantial amount of carrier. This is a particularly effective method when the physical nature of the sulfenylated acylhydrazone is such as to permit what is known as "low-volume" application; that is, when the compounds are in liquid form or substantially soluble in higher boiling solvents.

The application rate will, of course, vary depending upon the purpose for such application, the sulfenylated acylhydrazones being utilized, the frequency of dissemination and the like.

For use as agricultural bactericides and fungicides, dilute sprays can be applied at concentrations of from about 0.006 to about 2.397 kg of the active sulfenylated acylhydrazone ingredient per 100 liters of spray (about 0.05 to about 20 pounds of the active sulfenylated acylhydrazone ingredient per 100 gallons of spray). They are usually applied at from about 0.012 to about 1.2 kg per 100 liters (about 0.1 to about 10 pounds per 100 gallons) and preferably at from about 0.015 to about 0.599 kg per 100 liters (about 0.125 to about 5 pounds per 100 gallons). In more concentrated sprays, the active ingredient is increased by a factor of about 2 to 12. With dilute sprays, applications are usually made to the plants until run-off is achieved; whereas, with more concentrated or low-volume sprays, the materials are applied as mists.

The compounds of this invention may be utilized as the sole biocidal agents or they may be employed in conjunction with other fungicides, bactericides, algaecides, slimicides, insecticides, miticides, or with other comparable pesticides.

**Claims for the Contracting States: BE CH DE FR GB IT LI LU NL SE**

1. A compound of the formula

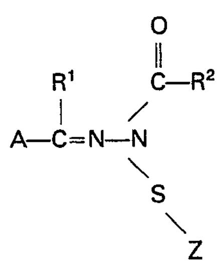

(I)

wherein

$R^1$ is hydrogen,
cyano,
straight or branched chain lower ($C_1$—$C_4$) alkyl optionally substituted with imidazolyl, triazolyl or one or more of the same or different halo; or
phenyl optionally substituted with one or more of the same or different halo, nitro, cyano, ($C_1$—$C_4$)-alkyl or ($C_1$—$C_4$)-alkoxy;

$R^2$ is hydrogen;
straight or branched chain ($C_1$—$C_{11}$)-alkyl optionally substituted with imidazolyl, triazolyl, or one or more of the same or different halo;
($C_1$—$C_4$)-alkoxy;
($C_1$—$C_4$)-thioalkoxy;
($C_2$—$C_6$)-alkenyl optionally substituted with one or more of the same or different halo;
heterocyclic optionally substituted with one or more of the same or different halo or nitro;
phenyl optionally substituted with one or more of the same or different halo;
($C_1$—$C_4$)-alkyl, ($C_1$—$C_4$)-alkoxy or nitro; or
phenalkyl where the alkyl moiety contains one to four carbon atoms and the phenyl ring is optionally substituted with one or more of the same or different halo (chloro, fluoro, bromo or iodo), cyano, nitro, ($C_1$—$C_4$)-alkyl, ($C_1$—$C_4$)-alkoxy, or ($C_1$—$C_4$)-haloalkyl;

Z is ($C_1$—$C_4$)-haloalkyl; and

A is phenyl optionally substituted with one or more of the same or different halo, nitro, cyano, hydroxy, ($C_1$—$C_6$)-alkyl, ($C_1$—$C_6$)-haloalkyl, ($C_1$—$C_6$)-thioalkyl, ($C_1$—$C_6$)-alkoxy, ($C_1$—$C_6$)-carbalkoxy, allyloxy,

($C_1$—$C_6$)-alkanoyloxy, ($C_1$—$C_6$)-alkylamino, ($C_1$—$C_6$)-dialkylamino or benzyloxy;
thienyl optionally substituted with one or more of the same or different nitro or halo;
furyl optionally substituted with one or more of the same or different nitro or halo;
pyridyl; or
naphthyl.

2. A compound according to Claim 1 wherein $R^2$ is other than substituted heterocyclic or substituted or unsubstituted phenalkyl and A is other than allyloxy substituted phenyl.

3. A compound according to claim 1 wherein:
$R^1$ is hydrogen, methyl or chloromethyl; and/or
$R^2$ is hydrogen, ($C_1$—$C_4$)-alkyl, ($C_1$—$C_4$)-haloalkyl, ($C_1$—$C_4$)-alkoxy or phenyl optionally substituted with one, two or three of the same or different halo, ($C_1$—$C_4$)-alkyl or ($C_1$—$C_4$)-alkoxy, and/or
Z is ($C_1$—$C_4$)-haloalkyl; and/or
A is phenyl optionally substituted with one, two or three of the same or different halo, nitro, cyano, trifluoromethyl, ($C_1$—$C_4$)-alkyl, ($C_1$—$C_4$)-alkoxy, ($C_1$—$C_4$)-carbalkoxy, or ($C_1$—$C_4$)-alkanoyloxy,
thienyl optionally substituted with nitro or halo; or
furyl.

4. A compound according to Claim 3 wherein
$R^1$ is hydrogen or methyl; and/or
$R^2$ is hydrogen, ($C_1$—$C_4$)-alkoxy or halophenyl; and/or
Z is trichloromethyl; and/or
A is phenyl substituted with one, two or three of the same or different chloro, fluoro, bromo, nitro, cyano, trifluoromethyl, ($C_1$—$C_4$)-alkyl, ($C_1$—$C_4$)-alkoxy, ($C_1$—$C_4$)-carbalkoxy or ($C_1$—$C_4$)-alkanoyloxy; or
thienyl optionally substituted with nitro or halo; or
furyl.

5. A compound according to Claim 4 wherein:
$R^1$ is hydrogen;
$R^2$ is methoxy;
Z is trichloromethyl; and
A is 4-carbomethoxyphenyl or
3-methoxyphenyl or
2-fluorophenyl or
3-nitrophenyl or
2,6-dichlorophenyl or
2-chlorophenyl or
4 bromophenyl or
2 bromophenyl or
4 fluorophenyl or
4 cyanophenyl.

6. A compound according to Claim 4 wherein:
$R^1$ is hydrogen;
$R^2$ is hydrogen;
Z is trichloromethyl; and
A is 4-chlorophenyl or
2-chlorophenyl or
para-tolyl or
2-chloro-6-fluorophenyl or
2,6-dichlorophenyl or
4-trifluoromethylphenyl or
4-bromophenyl or
3-chlorophenyl or
2-fluorophenyl or
2,4-dichlorophenyl or
3-bromophenyl or
2,6-difluorophenyl or
5-bromo-2-thienyl or
2-methoxyphenyl or
3-methoxyphenyl or
4-methoxyphenyl or
2,4-dimethylbenzyl or
2,5-dimethylbenzyl or
3-nitrophenyl or
4-carbmethoxyphenyl or
4-acetoxyphenyl.

7. The compound according to Claim 4 wherein:

$R^1$ is hydrogen;
$R^2$ is 2-chlorophenyl;
Z is trichloromethyl; and
A is 4-chlorophenyl.

8. A bactericidal and/or fungicidal composition comprising a compound according to any one of the preceding claims and an environmentally, preferably an agronomically acceptable carrier or diluent for the active bactericidal and/or fungicidal ingredient.

9. A method of controlling bacteria or fungi which comprises applying directly to bacteria or fungi or to the locus to be freed or protected from attack by bacteria or fungi, a bactericidally or fungicidally effective amount of a compound according to any one of claims 1 to 7 optionally in a composition according to Claim 8.

10. A process for producing a compound according to Claim 1 comprising reacting substantially equimolar quantities of a hydrazone of the formula

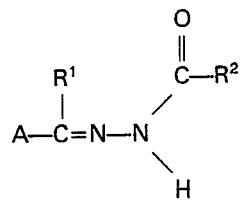

(II)

with a sulfenylating agent of the formula

$$Hal—S—Z \quad (III)$$

in an inert solvent or solvent mixture and in the presence of a base at a temperature of about 0°C to about 110°C to obtain a sulfenylated acylhydrazone and optionally isolating the sulfenylated acylhydrazone so produced or converting it to another compound according to Claim 1; $R^1$, $R^2$, A and Z being as defined in Claim 1 and Hal being halogen.

11. A process according to Claim 10 wherein the hydrazone of formula II has been prepared by reacting substantially equimolar quantities of a carbonyl of the formula

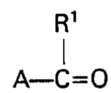

(IV)

with a carbonyl hydrazide of the formula

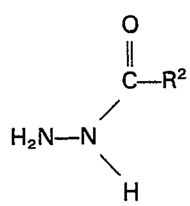

(V)

in the presence of inert solvent at a temperature of 20°C to 100°C.

**Claims for the Contracting State: AT**

1. A bactericidal and/or fungicidal composition comprising a compound of the formula

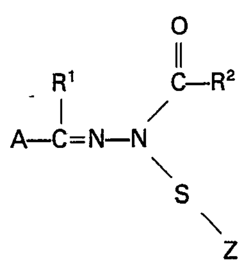

(I)

wherein

$R^1$ is hydrogen,
cyano,
straight or branched chain lower ($C_1$—$C_4$) alkyl optionally substituted with imidazolyl, triazolyl or one or more of the same or different halo; or
phenyl optionally substituted with one or more of the same or different halo, nitro, cyano, ($C_1$—$C_4$)-alkyl or ($C_1$—$C_4$)-alkoxy;

$R^2$ is hydrogen;
straight or branched chain ($C_1$—$C_{11}$)-alkyl optionally substituted with imidazolyl, triazolyl, or one or more of the same or different halo;
($C_1$—$C_4$)-alkoxy;
($C_1$—$C_4$)-thioalkoxy;
($C_2$—$C_6$)-alkenyl optionally substituted with one or more of the same or different halo;
heterocyclic optionally substituted with one or more of the same or different halo or nitro;
phenyl optionally substituted with one or more of the same or different halo;
($C_1$—$C_4$)-alkyl, ($C_1$—$C_4$)-alkoxy or nitro; or
phenalkyl where the alkyl moiety contains one to four carbon atoms and the phenyl ring is optionally substituted with one or more of the same or different halo (chloro, fluoro, bromo or iodo), cyano, nitro, ($C_1$—$C_4$)-alkyl, ($C_1$—$C_4$)-alkoxy, or ($C_1$—$C_4$)-haloalkyl;

Z is ($C_1$—$C_4$)-haloalkyl; and

A is phenyl optionally substituted with one or more of the same or different halo, nitro, cyano, hydroxy, ($C_1$—$C_6$)-alkyl, ($C_1$—$C_6$)-haloalkyl, ($C_1$—$C_6$)-thioalkyl, ($C_1$—$C_6$)-alkoxy, ($C_1$—$C_6$)-carbalkoxy, allyloxy, ($C_1$—$C_6$)-alkanoyloxy, ($C_1$—$C_6$)-alkylamino, ($C_1$—$C_6$)-dialkylamino or benzyloxy;
thienyl optionally substituted with one or more of the same or different nitro or halo;
furyl optionally substituted with one or more of the same or different nitro or halo;
pyridyl; or
naphthyl;

and an environmentally preferably an agronomically, acceptable diluent or carrier for the active bactericidal or fungicidal ingredient.

2. A composition according to Claim 1 wherein $R^2$ is other than substituted heterocyclic or substituted or unsubstituted phenalkyl and A is other than allyloxy substituted phenyl.

3. A composition according to claim 1 wherein:

$R^1$ is hydrogen, methyl or chloromethyl; and/or

$R^2$ is hydrogen, ($C_1$—$C_4$)-alkyl, ($C_1$—$C_4$)-haloalkyl, ($C_1$—$C_4$)-alkoxy or phenyl optionally substituted with one, two or three of the same or different halo, ($C_1$—$C_4$)-alkyl or ($C_1$—$C_4$)-alkoxy, and/or

Z is ($C_1$—$C_4$)-haloalkyl; and/or

A is phenyl optionally substituted with one, two or three of the same or different halo, nitro, cyano, trifluoromethyl, ($C_1$—$C_6$)-alkyl, ($C_1$—$C_6$)-alkoxy, ($C_1$—$C_6$)-carbalkoxy, or ($C_1$—$C_6$)-alkanoyloxy,
thienyl optionally substituted with nitro or halo; or
furyl.

4. A composition according to Claim 3 wherein

$R^1$ is hydrogen or methyl; and/or

$R^2$ is hydrogen, ($C_1$—$C_4$)-alkoxy or halophenyl; and/or

Z is trichloromethyl; and/or

A is phenyl substituted with one, two or three of the same or different chloro, fluoro, bromo, nitro, cyano, trifluoromethyl, ($C_1$—$C_4$)-alkyl, ($C_1$—$C_4$)-alkoxy, ($C_1$—$C_4$)-carbalkoxy or ($C_1$—$C_4$)-alkanoyloxy; or
thienyl optionally substituted with nitro or halo; or
furyl.

5. A composition according to Claim 4 wherein:

$R^1$ is hydrogen;

$R^2$ is methoxy;

Z is trichloromethyl; and

A is 4-carbomethoxyphenyl or
3-methoxyphenyl or
2-fluorophenyl or
3-nitrophenyl or
2,6-dichlorophenyl or
2-chlorophenyl or
4 bromophenyl or
2 bromophenyl or
4 fluorophenyl or
4 cyanophenyl.

6. A composition according to Claim 4 wherein:

$R^1$ is hydrogen;

$R^2$ is hydrogen;
Z is trichloromethyl; and
A is 4-chlorophenyl or
2-chlorophenyl or
para-tolyl or
2-chloro-6-fluorophenyl or
2,6-dichlorophenyl or
4-trifluoromethylphenyl or
4-bromophenyl or
3-chlorophenyl or
2-fluorophenyl or
2,4-dichlorophenyl or
3-bromophenyl or
2,6-difluorophenyl or
5-bromo-2-thienyl or
2-methoxyphenyl or
3-methoxyphenyl or
4-methoxyphenyl or
2,4-dimethylbenzyl or
2,5-dimethylbenzyl or
3-nitrophenyl or
4-carbmethoxyphenyl or
4-acetoxyphenyl.

7. The composition according to Claim 4 wherein:
$R^1$ is hydrogen;
$R^2$ is 2-chlorophenyl;
Z is trichloromethyl; and
A is 4-chlorophenyl.

8. A method of controlling bacteria or fungi which comprises applying directly to bacteria or fungi or to the locus to be freed or protected from attack by bacteria or fungi, a bactericidally or fungicidally effective amount of a compound as defined in any one of claims 1 to 7 optionally in a composition according to any preceding claim.

9. A process for producing a compound according to Claim 1 comprising reacting substantially equimolar quantities of a hydrazone of the formula

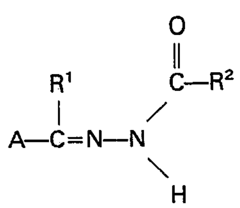

(II)

with a sulfenylating agent of the formula

Hal—S—Z (III)

in an inert solvent or solvent mixture and in the presence of a base at a temperature of about 0°C to about 110°C to obtain a sulfenylated acylhydrazone and optionally isolating the sulfenylated acylhydrazone so produced or converting it to another compound according to Claim 1; $R^1$, $R^2$, A and Z being as defined in Claim 1 and Hal being halogen.

10. A process according to Claim 9 wherein the hydrazone of formula II has been prepared by reacting substantially equimolar quantities of a carbonyl of the formula

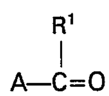

(IV)

with a carbonyl hydrazide of the formula

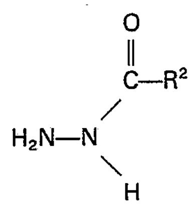

(V)

in the presence of inert solvent at a temperature of 20°C to 100°C.

**Patentansprüche für die Vertragsstaaten: BE CH DE FR GB IT LI LU NL SE**

1. Verbindung der Formel

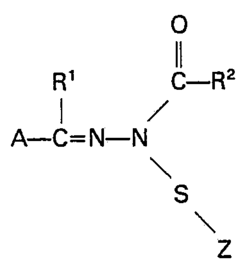

(I)

worin bedeuten

$R^1$ Wasserstoff,
Cyano,
geradkettiges oder verzweigtes niederes $(C_1-C_4)$Alkyl, gegebenenfalls substituiert mit Imidazolyl, Triazolyl oder einem oder mehreren gleichen oder verschiedenen Substituenten, ausgewählt aus Halogen oder Phenyl, gegebenenfalls substituiert mit einem oder mehreren gleichen oder verschiedenen Substituenten, ausgewählt aus Halogen, Nitro, Cyano, $(C_1-C_4)$Alkyl oder $(C_1-C_4)$Alkoxy,

$R^2$ Wasserstoff,
geradkettiges oder verzweigtes $(C_1-C_{11})$Alkyl, gegebenenfalls substituiert mit Imidazolyl, Triazolyl oder einem oder mehreren gleichen oder verschiedenen Substituenten, ausgewählt aus Halogenen,
$(C_1-C_4)$Alkoxy,
$(C_1-C_4)$Thioalkoxy,
$(C_2-C_6)$Alkenyl, gegebenenfalls substituiert mit einem oder mehreren gleichen oder verschiedenen Substituenten, ausgewählt aus Halogenen, Heterozyklen, gegebenenfalls substituiert mit einem oder mehreren gleichen oder verschiedenen Substituenten, ausgewählt aus Halgenen oder Nitro,
Phenyl, gegebenenfalls substituiert mit einem oder mehreren gleichen oder verschiedenen Substituenten, ausgewählt aus Halogenen, $(C_1-C_4)$Alkyl, $(C_1-C_4)$Alkoxy oder Nitro, oder Phenalkyl, wobei der Alkylanteil 1 bis 4 Kohlenstoffatome enthält und der Phenylring gegebenenfalls mit einem oder mehreren gleichen oder verschiedenen Substituenten substituiert ist, ausgewählt aus Halogenen (Chlor, Fluor, Brom oder Jod), Cyano, Nitro, $(C_1-C_4)$Alkyl, $(C_1-C_4)$Alkoxy oder $(C_1-C_4)$Halogenalkyl,

Z $(C_1-C_4)$Halogenalkyl und

A Phenyl, gegebenenfalls substituiert mit einem oder mehreren gleichen oder verschiedenen Substituenten, ausgewählt aus Halogen, Nitro, Cyano, Hydroxy, $(C_1-C_6)$Alkyl, $(C_1-C_6)$Halogenalkyl, $(C_1-C_6)$Thioalkyl, $(C_1-C_6)$Alkoxy, $(C_1-C_6)$Carbalkoxy, Allyloxy, $(C_1-C_6)$Alkanoyloxy, $(C_1-C_6)$Alkylamiono, $(C_1-C_6)$Dialkylamino oder Benzyloxy,
Thienyl, gegebenenfalls substituiert mit einem oder mehreren gleichen oder verschiedenen Substituenten, ausgewählt aus Nitro oder Halogenen,
Furyl, gegebenenfalls substituiert mit einem oder mehreren gleichen oder verschiedenen Substituenten, ausgewählt aus Nitro oder Halogenen,
Pyridyl oder
Naphthyl.

2. Verbindung nach Anspruch 1, worin $R^2$ eine andere Bedeutung hat als einen substituierten Heterozyklus oder substituiertes oder nichtsubstituiertes Phenalkyl und A eine andere Bedeutung hat als Allyloxy-substituiertes Phenyl.

3. Verbindung nach Anspruch 1, worin

$R^1$ Wasserstoff, Methyl oder Chlormethyl ist und/oder

$R^2$ Wasserstoff, $(C_1-C_4)$Alkyl, $(C_1-C_4)$Halogenalkyl, $(C_1-C_4)$Alkoxy oder Phenyl ist, gegebenenfalls substituiert mit 1, 2 oder 3 gleichen oder verschiedenen Substituenten, ausgewählt aus Halogen, $(C_1-C_4)$Alkyl oder $(C_1-C_4)$Alkoxy, und/oder

Z $(C_1—C_4)$Halogenalkyl ist, und/oder
A Phenyl ist, gegebenenfalls substituiert mit 1, 2 oder 3 gleichen oder verschiedenen Substituenten, ausgewählt aus Halogen, Nitro, Cyano, Trifluormethyl, $(C_1—C_6)$Alkyl, $(C_1—C_6)$Alkoxy, $(C_1—C_6)$Carbalkoxy oder $(C_1—C_6)$Alkanoyloxy,
Thienyl, gegebenenfalls substituiert mit Nitro oder Halogen, oder
Furyl ist.

4. Verbindung nach Anspruch 3, worin
$R^1$ Wasserstoff oder Methyl ist, und/oder
$R^2$ Wasserstoff, $(C_1—C_4)$Alkoxy oder Halogenphenyl ist, und/oder
Z Trichlormethyl ist, und/oder
A Phenyl, substituiert mit 1, 2 oder 3 gleichen oder verschiedenen Substituenten, ausgewählt aus Chlor, Fluor, Brom, Nitro, Cyano, Trifluormethyl, $(C_1—C_4)$Alkyl, $(C_1—C_4)$Alkoxy, $(C_1—C_4)$Carbalkoxy oder $(C_1—C_4)$Alkanoyloxy, oder
Thienyl, gegebenenfalls substituiert mit Nitro oder Halogen, oder
Furyl ist.

5. Verbindung nach Anspruch 4, worin
$R^1$ Wasserstoff ist,
$R^2$ Methoxy ist,
Z Trichlormethyl ist, und
A 4-Carbomethoxyphenyl oder
3-Methoxyphenyl oder
2-Fluorphenyl oder
3-Nitrophenyl oder
2,6-Dichlorphenyl oder
2-Chlorphenyl oder
4-Bromphenyl oder
2-Bromphenyl oder
4-Fluorphenyl oder
4-Cyanophenyl ist.

6. Verbindung nach Anspruch 4, worin
$R^1$ Wasserstoff ist,
$R^2$ Wasserstoff ist,
Z Trichlormethyl ist, und
A 4-Chlorphenyl oder
2-Chlorphenyl oder
p-Tolyl oder
2-Chlor-6-fluorphenyl oder
2,6-Dichlorphenyl oder
4-Trifluormethylphenyl oder
4-Bromphenyl oder
3-Chlorphenyl oder
2-Fluorphenyl oder
2,4-Dichlorphenyl oder
3-Bromphenyl oder
2,6-Difluorphenyl oder
5-Brom-2-thienyl oder
2-Methoxyphenyl oder
3-Methoxyphenyl oder
4-Methoxyphenyl oder
2,4-Dimethylbenzyl oder
2,5-Dimethylbenzyl oder
3-Nitrophenyl oder
4-Carbomethoxyphenyl oder
4-Acetoxyphenyl ist.

7. Verbindung nach Anspruch 4, worin
$R^1$ Wasserstoff ist,
$R^2$ 2-Chlorphenyl ist,
Z Trichlormethyl ist, und
A 4-Chlorphenyl ist.

8. Bakterizides und/oder fungizides Mittel aus einer Verbindung gemäß einem der vorhergehenden Ansprüche und einem für die Umgebung verträglichen Träger oder Verdünnungsmittel, insbesondere einem für landwirtschaftliche Zwecke verträglichen Träger oder Verdünnungsmittel, für den aktiven bakteriziden und/oder fungiziden Bestandteil.

9. Verfahren zur Bekämpfung von Bakterien oder Pilzen, wobei direkt auf die Bakterien oder Pilze oder auf die Stelle, die von Bakterien oder Pilzen zu befreien oder gegenüber einem Angriff durch dieselben zu

schützen ist, eine bakterizid oder fungizid wirksame Menge einer Verbindung gemäß einem der Ansprüche 1 bis 7, gegebenenfalls in einem Mittel gemäß Anspruch 8, aufgebracht wird.

10. Verfahren zur Herstellungt einer Verbindung gemäß Anspruch 1, wobei im wesentlichen äquimolare Mengen eines Hydrazons der Formel

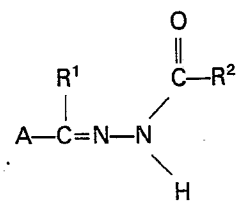

(II)

mit einem Sulfenylierungsmittel der Formel

Hal—S—Z (III)

in einem inerten Lösungsmittel oder einer Lösungsmittelmischung sowie in Gegenwart einer Base bei einer Temperatur von ungefähr 0°C bis ungefähr 110°C zur Gewinnung eines sulfenylierten Acylhydrazons umgesetzt werden und gegebenenfalls das auf diese Weise erzeugte sulfenylierte Acylhydrazon isoliert oder in eine andere Verbindung gemäß Anspruch 1 umgewandelt wird, wobei $R^1$, $R^2$, A und Z die in Anspruch 1 angegebenen Bedeutungen besitzen und Hal ein Halogen ist.

11. Verfahren nach Anspruch 10, wobei das Hydrazon der Formel II hergestellt worden ist durch Umsetzung von im wesentlichen äquimolaren Mengen eines Carbonyls der Formel

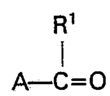

(IV)

mit einem Carbonylhydrazid der Formel

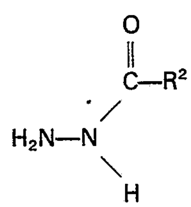

(V)

in Gegenwart eines inerten Lösungsmittels bei einer Temperatur von 20°C bis 100°C.

**Patentansprüche für den Vertragsstaat: AT**

1. Bakterizides und/oder fungizides Mittel aus einer Verbindung der Formel

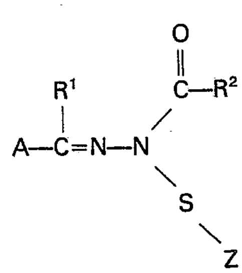

(I)

worin bedeuten

$R^1$ Wasserstoff,
Cyano,
geradkettiges oder verzweigtes niederes $(C_1—C_4)$Alkyl, gegebenenfalls substituiert mit Imidazolyl, Triazolyl oder einem oder mehreren gleichen oder verschiedenen Substituenten, ausgewählt aus Halogen oder Phenyl, gegebenenfalls substituiert mit einem oder mehreren gleichen oder verschiedenen Substituenten, ausgewählt aus Halogen, Nitro, Cyano, $(C_1—C_4)$Alkyl oder $(C_1—C_4)$Alkoxy,

$R^2$ Wasserstoff,
geradkettiges oder verzweigtes ($C_1$—$C_{11}$)Alkyl, gegebenenfalls substituiert mit Imidazolyl, Triazolyl oder einem oder mehreren gleichen oder verschiedenen Substituenten, ausgewählt aus Halogenen,
($C_1$—$C_4$)Alkoxy,
($C_1$—$C_4$)Thioalkoxy,
($C_2$—$C_6$)Alkenyl, gegebenenfalls substituiert mit einem oder mehreren gleichen oder verschiedenen Substituenten, ausgewählt aus Halogenen, Heterozyklen, gegebenenfalls substituiert mit einem oder mehreren gleichen oder verschiedenen Substituenten, ausgewählt aus Halgenen oder Nitro,
Phenyl, gegebenenfalls substituiert mit einem oder mehreren gleichen oder verschiedenen Substituenten, ausgewählt aus Halogenen, ($C_1$—$C_4$)Alkyl, ($C_1$—$C_4$)Alkoxy oder Nitro, oder Phenalkyl, wobei der Alkylanteil 1 bis 4 Kohlenstoffatome enthält und der Phenylring gegebenenfalls mit einem oder mehreren gleichen oder verschiedenen Substituenten substituiert ist, ausgewählt aus Halogenen (Chlor, Fluor, Brom oder Jod), Cyano, Nitro, ($C_1$—$C_4$)Alkyl, ($C_1$—$C_4$)Alkoxy oder ($C_1$—$C_4$)Halogenalkyl,
Z ($C_1$—$C_4$)Halogenalkyl und
A Phenyl, gegebenenfalls substituiert mit einem oder mehreren gleichen oder verschiedenen Substituenten, ausgewählt aus Halogen, Nitro, Cyano, Hydroxy, ($C_1$—$C_6$)Alkyl, ($C_1$—$C_6$)Halogenalkyl, ($C_1$—$C_6$)Thioalkyl, ($C_1$—$C_6$)Alkoxy, ($C_1$—$C_6$)Carbalkoxy, Allyloxy, ($C_1$—$C_6$)Alkanoyloxy, ($C_1$—$C_6$)Alkylamiono, ($C_1$—$C_6$)Dialkylamino oder Benzyloxy,
Thienyl, gegebenenfalls substituiert mit einem oder mehreren gleichen oder verschiedenen Substituenten, ausgewählt aus Nitro oder Halogenen,
Furyl, gegebenenfalls substituiert mit einem oder mehreren gleichen oder verschiedenen Substituenten, ausgewählt aus Nitro oder Halogenen,
Pyridyl oder
Naphthyl,

und einem für die Umgebung verträglichen Verdünnungsmittel oder Träger, vorzugsweise einem für landwirtschaftliche Zwecke verträglichen Verdünnungsmittel oder Träger, für den aktiven bakteriziden oder fungiziden Bestandteil.

2. Mittel nach Anspruch 1, worin $R^2$ eine andere Bedeutung hat als ein substituierter Heterozyklus oder substituiertes oder nichtsubstituiertes Phenalkyl, und A eine andere Bedeutung hat als Allyloxy-substituiertes Phenyl.

3. Mittel nach Anspruch 1, worin
$R^1$ Wasserstoff, Methyl oder Chlormethyl ist und/oder
$R^2$ Wasserstoff, ($C_1$—$C_4$)Alkyl, ($C_1$—$C_4$)Halogenalkyl, ($C_1$—$C_4$)Alkoxy oder Phenyl, gegebenenfalls substituiert mit 1, 2 oder 3 gleichen oder verschiedenen Substituenten, ausgewählt aus Halogen, ($C_1$—$C_4$)Alkyl oder ($C_1$—$C_4$)Alkoxy, ist und/oder
Z ($C_1$—$C_4$)Halogenalkyl ist, und/oder
A Phenyl ist, gegebenenfalls substituiert mit 1, 2 oder 3 gleichen oder verschiedenen Substituenten, ausgewählt aus Halogen, Nitro, Cyano, Trifluormethyl, ($C_1$—$C_6$)Alkyl, ($C_1$—$C_6$)Alkoxy, ($C_1$—$C_6$)Carbalkoxy oder ($C_1$—$C_6$)Alkanoyloxy,
Thienyl, gegebenenfalls substituiert mit Nitro oder Halogen, oder
Furyl ist.

4. Mittel nach Anspruch 3, worin
$R^1$ Wasserstoff oder Methyl ist, und/oder
$R^2$ Wasserstoff, ($C_1$—$C_4$)Alkoxy oder Halogenphenyl ist, und/oder
Z Trichlormethyl ist, und/oder
A Phenyl, substituiert mit 1, 2 oder 3 gleichen oder verschiedenen Substituenten, ausgewählt aus Chlor, Fluor, Brom, Nitro, Cyano, Trifluormethyl, ($C_1$—$C_4$)Alkyl, ($C_1$—$C_4$)Alkoxy, ($C_1$—$C_4$)Carbalkoxy oder ($C_1$—$C_4$)Alkanoyloxy, oder
Thienyl, gegebenenfalls substituiert mit Nitro oder Halogen, oder
Furyl ist.

5. Mittel nach Anspruch 4, worin
$R^1$ Wasserstoff ist,
$R^2$ Methoxy ist,
Z Trichlormethyl ist, und
A 4-Carbomethoxyphenyl oder
3-Methoxyphenyl oder
2-Fluorphenyl oder
3-Nitrophenyl oder
2,6-Dichlorphenyl oder
2-Chlorphenyl oder
4-Bromphenyl oder
2-Bromphenyl oder
4-Fluorphenyl oder
4-Cyanophenyl ist.

6. Mittel nach Anspruch 4, worin
$R^1$ Wasserstoff ist,
$R^2$ Wasserstoff ist,
Z Trichlormethyl ist, und
A 4-Chlorphenyl oder
2-Chlorphenyl oder
p-Tolyl oder
2-Chlor-6-fluorphenyl oder
2,6-Dichlorphenyl oder
4-Trifluormethylphenyl oder
4-Bromphenyl oder
3-Chlorphenyl oder
2-Fluorphenyl oder
2,4-Dichlorphenyl oder
3-Bromphenyl oder
2,6-Difluorphenyl oder
5-Brom-2-thienyl oder
2-Methoxyphenyl oder
3-Methoxyphenyl oder
4-Methoxyphenyl oder
2,4-Dimethylbenzyl oder
2,5-Dimethylbenzyl oder
3-Nitrophenyl oder
4-Carbomethoxyphenyl oder
4-Acetoxyphenyl ist.

7. Mittel nach Anspruch 4, worin
$R^1$ Wasserstoff ist,
$R^2$ 2-Chlorphenyl ist,
Z Trichlormethyl ist, und
A 4-Chlorphenyl ist.

8. Verfahren zur Bekämpfung von Bakterien oder Pilzen, wobei direkt auf Bakterien oder Pilze oder auf die von Bakterien oder Pilzen zu befreiende Stelle oder vor diesen zu schützende Stelle eine bakterizid oder fungizid wirksame Menge einer Verbindung gemäß einem der Ansprüche 1 bis 7, gegebenenfalls in einem Mittel gemäß einem der vorhergehenden Ansprüche, aufgebracht wird.

9. Verfahren zur Herstellung einer Verbindung gemäß Anspruch 1 durch Umsetzung im wesentlichen äquimolarer Mengen eines Hydrazons der Formel

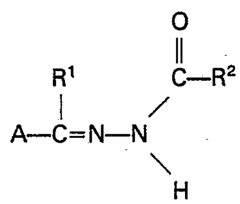

(II)

mit einem Sulfenylierungsmittel der Formel

Hal—S—Z (III)

in einem inerten Lösungsmittel oder einer Lösungsmittelmischung sowie in Gegenwart einer Base bei einer Temperatur von ungefähr 0°C bis ungefähr 110°C zur Gewinnung eines sulfenylierten Acylhydrazons und gegebenenfalls Isolieren des auf diese Weise erzeugten sulfenylierten Acylhydrazons oder Umwandeln desselben in eine andere Verbindung gemäß Anspruch 1, wobei $R^1$, $R^2$, A und Z die in Anspruch 1 angegebene Bedeutungen besitzen und Hal Halogen ist.

10. Verfahren nach Anspruch 9, wobei das Hydrazon der Formel II hergestellt worden ist durch Umsetzung von im wesentlichen äquimolaren Mengen eines Carbonyls der Formel

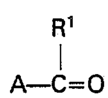

(IV)

mit einem Carbonylhydrazid der Formel

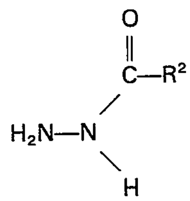

(V)

in Gegenwart eines inerten Lösungsmittels bei einer Temperatur von 20°C bis 100°C.

**Revendications pour les Etats contractants: BE CH DE FR GB IT LI LU NL SE**

1. Un composé de formule

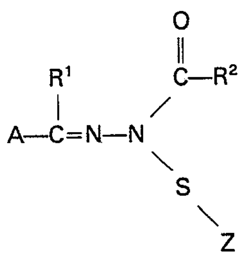

(I)

dans laquelle

$R^1$ est un hydrogène, un cyano, un alcoyle inférieur ($C_1$—$C_4$) à chaîne droite ou ramifiée facultativement substitué par un imidazolyle, un triazolyle ou un ou plusieurs halogéno semblables ou différents; ou un phényle, facultativement substitué par un ou plusieurs halogéno, nitro, cyano, alcoyles ($C_1$—$C_4$) ou alcoxy ($C_1$—$C_4$) semblables ou différents;

$R^2$ est un hydrogène; un alcoyle ($C_1$—$C_{11}$) à chaîne droite ou ramifiée, facultativement substitué par un imidazolyle, un triazolyle ou un ou plusieurs halogéno semblables ou différents; un alcoxy ($C_1$—$C_4$); un thioalcoxy ($C_1$—$C_4$); un alcényle ($C_2$—$C_6$) facultativement substitué par un ou plusieurs halogéno semblables ou différents; un hétérocycle facultativement substitué par un ou plusieurs halogéno ou nitro semblables ou différents; un phényle facultativement substitué par un ou plusieurs halogéno, alcoyles ($C_1$—$C_4$), alcoxy ($C_1$—$C_4$) ou nitro semblables ou différents; ou un phénalcoyle dont le fragment alcoyle contient 1 à 4 atomes de carbone et le cycle phényle est facultativement substitué par un ou plusieurs halogéno (chloro, fluoro, bromo ou iodo), cyano, nitro, alcoyles ($C_1$—$C_4$), alcoxy ($C_1$—$C_4$) ou halogénoalcoyles ($C_1$—$C_4$) semblables ou différents;

Z est un halogénoalcoyle ($C_1$—$C_4$); et

A est un phényle facultativement substitué par un ou plusieurs halogéno, nitro, cyano, hydroxy, alcoyles ($C_1$—$C_6$), halogénoalcoyles ($C_1$—$C_6$), thioalcoyles ($C_1$—$C_6$), alcoxy ($C_1$—$C_6$), carbalcoxy ($C_1$—$C_6$), allyloxy, alcanoyloxy ($C_1$—$C_6$), alcoylamino ($C_1$—$C_6$), di-alcoyl($C_1$—$C_6$)amino ou benzyloxy semblables ou différents; un thiényle facultativement substitué par un ou plusieurs nitro ou halogéno semblables ou différents; un furyle facultativement substitué par un ou plusieurs nitro ou halogéno semblables ou différents; un pyridyle; ou un naphtyle.

2. Un composé selon la revendication 1 dans lequel $R^2$ est autre qu'un hétérocycle substitué ou un phénalcoyle substitué ou non substitué et A est autre qu'un phényle allyloxy-substitué.

3. Un composé selon la revendication 1 où:

$R^1$ est un hydrogène, un méthyle ou un chlorométhyle; et/ou

$R^2$ est un hydrogène, un alcoyle ($C_1$—$C_4$), un halogénoalcoyle ($C_1$—$C_4$), un alcoxy ($C_1$—$C_4$) ou un phényle facultativement substitué par un, deux ou trois halogéno, alcoyles ($C_1$—$C_4$) ou alcoxy ($C_1$—$C_4$) semblables ou différents et/ou

Z est un halgénoalcoyle ($C_1$—$C_4$); et/ou

A est un phényle facultativement substitué par un, deux ou trois halogéno, nitro, cyano, trifluorométhyles, alcoyles ($C_1$—$C_6$), alcoxy ($C_1$—$C_6$), carbalcoxy ($C_1$—$C_6$) ou alcanoyloxy ($C_1$—$C_6$) semblables ou différents,

un thiényle facultativement substitué par nitro ou halogéno; ou
un furyle.

4. Un composé selon la revendication 3 où:

$R^1$ est un hydrogène ou un méthyle et/ou
$R^2$ est un hydrogène, un alcoxy ($C_1$—$C_4$) ou un halogénophényle; et/ou
Z est un trichlorométhyle; et/ou
A est un phényle substitué par un, deux ou trois chloro, fluoro, bromo, nitro, cyano, trifluorométhyles, alcoyles ($C_1$—$C_4$), alcoxy ($C_1$—$C_4$), carbalcoxy ($C_1$—$C_4$) ou alcanoyloxy ($C_1$—$C_4$) semblables ou différents; ou
un thiényle facultativement substitué par nitro ou halogéno; ou
un furyle.

5. Un composé selon la revendication 4 où:

$R^1$ est un hydrogène;
$R^2$ est un méthoxy;
Z est un trichlorométhyle; et
A est un 4-carbométhoxyphényle ou
un 3-méthoxyphényle ou
un 2-fluorophényle ou
un 3-nitrophényle ou
un 2,6-dichlorophényle ou
un 2-chlorophényle ou
un 4-bromophényle ou
un 2-bromophényle ou
un 4-fluorophényle ou
un 4-cyanophényle.

6. Un composé selon la revendication 4 où:

$R^1$ est un hydrogène;
$R^2$ est un hydrogène;
Z est un trichlorométhyle; et
A est un 4-chlorophényle ou
un 2-chlorophényle ou
un para-tolyle ou
un 2-chloro-6-fluorophényle ou
un 2,6-dichlorophényle ou
un 4-trifluorométhylphényle ou
un 4-bromophényle ou
un 3-chlorophényle ou
un 2-fluorophényle ou
un 2,4-dichlorophényle ou
un 3-bromophényle ou
un 2,6-difluorophényle ou
un 5-bromo-2-thiényle ou
un 2-méthoxyphényle ou
un 3-méthoxyphényle ou
un 4-méthoxyphényle ou
un 2,4-diméthylbenzyle ou
un 2,5-diméthylbenzyle ou
un 3-nitrophényle ou
un 4-carbométhoxyphényle ou
un 4-acétoxyphényle.

7. Le composé selon la revendication 4 où:

$R^1$ est un hydrogène;
$R^2$ est un 2-chlorophényle;
Z est un trichlorométhyle; et
A est un 4-chlorophényle.

8. Une composition bactéricide et/ou fongicide comprenant un composé selon l'une quelconque des revendications précédentes et un support ou diluant acceptable pour l'environnement, et de préférence convenant en agronomie, de l'ingrédient actif bactéricide et/ou fongicide.

9. Un procédé de lutte contre les bactéries ou les champignons qui comprend l'application directe aux bactéries ou aux champignons ou aux sites à débarrasser ou à protéger de l'attaque par les bactéries ou les champignons d'une quantité efficace bactéricide ou fongicide d'un composé selon l'une quelconque des revendications 1 à 7 facultativement dans une composition selon la revendication 8.

10. Un procédé pour la production d'un composé selon la revendication 1 comprenant la réaction de quantités pratiquement équimoléculaires d'une hydrazone de formule

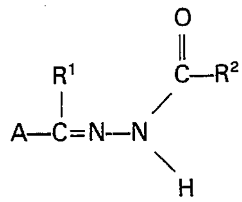

(II)

avec un agent de sulfénylation de formule

Hal—S—Z (III)

dans un solvant ou un mélange solvant inertes et en présence d'une base à une température d'environ 0°C à environ 110°C, pour obtenir une acylhydrazone sulfénylée, et facultativement l'isolement de l'acylhydrazone sulfénylée ainsi produite ou sa conversion en un autre composé selon la revendication 1; $R^1$, $R^2$, A et Z étant comme défini dans la revendication 1 et Hal étant un halogène.

11. Un procédé selon la revendication 10 dans lequel l'hydrazone de formule II a été préparée par réaction de quantités pratiquement équimoléculaires d'un carbonyle de formule

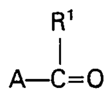

(IV)

avec un carbonylhydrazide de formule

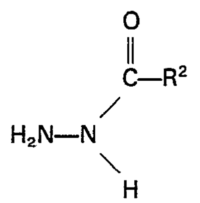

(V)

en présence d'un solvant inerte à une température de 20°C à 100°C.

**Revendications pour l'Etat contractant: AT**

1. Une composition bactéricide et/ou fongicide comprenant un composé de formule

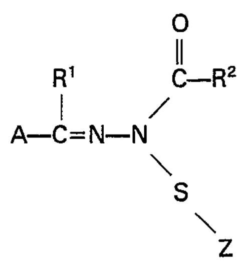

(I)

dans laquelle

$R^1$ est un hydrogène,
un cyano,
un alcoyle inférieur ($C_1$—$C_4$) à chaîne droite ou ramifiée facultativement substitué par un imidazolyle, un triazolyle ou un ou plusieurs halogéno semblables ou différents;
ou un phényle, facultativement substitué par un ou plusieurs halogéno, nitro, cyano, alcoyles ($C_1$—$C_4$) ou alcoxy ($C_1$—$C_4$) semblables ou différents;

$R^2$ est un hydrogène;
un alcoyle ($C_1$—$C_{11}$) à chaîne droite ou ramifiée, facultativement substitué par un imidazolyle, un triazolyle ou un ou plusieurs halogéno semblables ou différents;
un alcoxy ($C_1$—$C_4$);
un thioalcoxy ($C_1$—$C_4$);
un alcényle ($C_2$—$C_6$) facultativement substitué par un ou plusieurs halgéno semblables ou différents;
un hétérocycle facultativement substitué par un ou plusieurs halogéno ou nitro semblables ou différents;

un phényle facultativement substitué par un ou plusieurs halogéno, alcoyles ($C_1$—$C_4$), alcoxy ($C_1$—$C_4$) ou nitro semblables ou différents;
ou un phénalcoyle dont le fragment alcoyle contient 1 à 4 atomes de carbone et le cycle phényle est facultativement substitué par un ou plusieurs halogéno (chloro, fluoro, bromo ou iodo), cyano, nitro, alcoyles ($C_1$—$C_4$), alcoxy ($C_1$—$C_4$) ou halogénoalcoyles ($C_1$—$C_4$) semblables ou différents;

Z est un halogénoalcoyle ($C_1$—$C_4$); et

A est un phényle facultativement substitué par un ou plusieurs halogéno, nitro, cyano, hydroxy, alcoyles ($C_1$—$C_6$), halogénoalcoyles ($C_1$—$C_6$), thioalcoyles ($C_1$—$C_6$), alcoxy ($C_1$—$C_6$), carbalcoxy ($C_1$—$C_6$), allyloxy, alcanoyloxy ($C_1$—$C_6$), alcoylamino ($C_1$—$C_6$), di-alcoyl($C_1$—$C_6$)amino ou benzyloxy semblables ou différents;
un thiényle facultativement substitué par un ou plusieurs nitro ou halogéno semblables ou différents;
un furyle facultativement substitué par un ou plusieurs nitro ou halogéno semblables ou différents;
un pyridyle; ou
un naphtyle;

et un diluant ou support acceptables pour l'environnement, et de préférence convenant en agronomie, de l'ingrédient actif bactéricide ou fongicide.

2. Une composition selon la revendication 1 dans laquelle $R^2$ est autre qu'un hétérocycle substitué ou un phénalcoyle substitué ou non substitué et A est autre qu'un phényle allyloxy-substitué.

3. Une composition selon la revendication 1 où:

$R^1$ est un hydrogène, un méthyle ou un chlorométhyle; et/ou

$R^2$ est un hydrogène, un alcoyle ($C_1$—$C_4$), un halogénoalcoyle ($C_1$—$C_4$), un alcoxy ($C_1$—$C_4$) ou un phényle facultativement substitué par un, deux ou trois halogéno, alcoyles ($C_1$—$C_4$) ou alcoxy ($C_1$—$C_4$) semblables ou différents et/ou

Z est un halgénoalcoyle ($C_1$—$C_4$); et/ou

A est un phényl facultativement substitué par un, deux ou trois halogéno, nitro, cyano, trifluorométhyles, alcoyles ($C_1$—$C_6$), alcoxy ($C_1$—$C_6$), carbalcoxy ($C_1$—$C_6$) ou alcanoyloxy ($C_1$—$C_6$) semblables ou différents,
un thiényle facultativement substitué par nitro ou halogéno; ou
un furyle.

4. Une composition selon la revendication 3 où:

$R^1$ est un hydrogène ou un méthyle et/ou

$R^2$ est un hydrogène, un alcoxy ($C_1$—$C_4$) ou un halogénophényle; et/ou

Z est un trichlorométhyle; et/ou

A est un phényle substitué par un, deux ou trois chloro, fluoro, bromo, nitro, cyano, trifluorométhyles, alcoyles ($C_1$—$C_4$), alcoxy ($C_1$—$C_4$), carbalcoxy ($C_1$—$C_4$) ou alcanoyloxy ($C_1$—$C_4$) semblables ou différents; ou
un thiényle facultativement substitué par nitro ou halogéno; ou
un furyle.

5. Une composition selon la revendication 4 où:

$R^1$ est un hydrogène;

$R^2$ est un méthoxy;

Z est un trichlorométhyle; et

A est un 4-carbométhoxyphényle ou
un 3-méthoxyphényle ou
un 2-fluorophényle ou
un 3-nitrophényle ou
un 2,6-dichlorophényle ou
un 2-chlorophényle ou
un 4-bromophényle ou
un 2-bromophényle ou
un 4-fluorophényle ou
un 4-cyanophényle.

6. Une composition selon la revendication 4 où:

$R^1$ est un hydrogène;

$R^2$ est un hydrogène;

Z est un trichlorométhyle; et

A est un 4-chlorophényle ou
un 2-chlorophényle ou
un para-tolyle ou
un 2-chloro-6-fluorophényle ou
un 2,6-dichlorophényle ou
un 4-trifluorométhylphényle ou
un 4-bromophényle ou
un 3-chlorophényle ou
un 2-fluorophényle ou

un 2,4-dichlorophényle ou
un 3-bromophényle ou
un 2,6-difluorophényle ou
un 5-bromo-2-thiényle ou
un 2-méthoxyphényle ou
un 3-méthoxyphényle ou
un 4-méthoxyphényle ou
un 2,4-diméthylbenzyle ou
un 2,5-diméthylbenzyle ou
un 3-nitrophényle ou
un 4-carbométhoxyphényle ou
un 4-acétoxyphényle.

7. La composition selon la revendication 4 où:

$R^1$ est un hydrogène;
$R^2$ est un 2-chlorophényle;
Z est un trichlorométhyle; et
A est un 4-chlorophényle.

8. Un procédé de lutte contre les bactéries ou les champignons qui comprend l'application directe aux bactéries ou aux champignons ou aux sites à débarrasser ou à protéger de l'attaque par les bactéries ou les champignons d'une quantité efficace bactéricide ou fongicide d'un composé selon l'une quelconque des revendications 1 à 7 facultativement dans une composition selon l'une quelconque des revendications précédentes.

9. Un procédé pour la production d'un composé selon la revendication 1 comprenant la réaction de quantités pratiquement équimoléculaires d'une hydrazone de formule

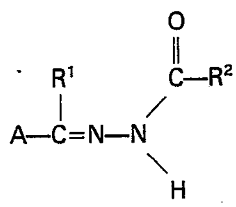

(II)

avec un agent de sulfénylation de formule

Hal—S—Z (III)

dans un solvant ou un mélange solvant inertes et en présence d'une base à une température d'environ 0°C à environ 110°C, pour obtenir une acylhydrazone sulfénylée, et facultativement l'isolement de l'acylhydrazone sulfénylée ainsi produite ou sa conversion en un autre composé selon la revendication 1; $R^1$, $R^2$, A et Z étant comme défini dans la revendication 1 et Hal étant un halogène.

10. Un procédé selon la revendication 9 dans lequel l'hydrazone de formule II a été préparée par réaction de quantités pratiquement équimoléculaires d'un carbonyle de formule

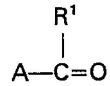

(IV)

avec un carbonylhydrazide de formule

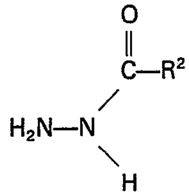

(V)

en présence d'un solvant inerte à une température de 20°C à 100°C.